# EUROPEAN PATENT SPECIFICATION

(11) **EP 1 831 351 B1**
(45) Date of publication and mention of the grant of the patent: **24.08.2011**
(21) Application number: 05817653.8
(22) Date of filing: 14.12.2005
(51) Int. Cl.: C12N 5/077, C12N 5/0797, C12N 5/0786, C12N 5/0789, C12N 5/10, C12N 5/16

(54) **PRODUCTION FROM BLOOD OF CELLS OF NEURAL LINEAGE**
HERSTELLUNG VON NEURALLINIENZELLEN AUS BLUT
PRODUCTION DE CELLULES DE LIGNEE NEURONALE A PARTIR DU SANG

(30) Priority: 14.12.2004 US 636391 P; 05.04.2005 US 668739 P
(43) Date of publication of application: 12.09.2007
(60) Divisional application: 10190450.6
(73) Proprietor: Kwalata Trading Limited, 1522 Nicosia (CY)
(72) Inventor: FULGA, Valentin, 62996 Tel Aviv (IL); PORAT, Yael, 45283 Hod Hasharon (IL); POROZOV, Svetlana, 76251 Rehovot (IL)
(74) Representative: Spencer, Matthew Peter
(86) International application number: PCT/IL2005/001348
(87) International publication number: WO 2006/064504

(56) References cited:
- WO-A-03/055989
- WO-A-03/078610
- US-A1- 2002 151 056
- LIOUTERMAN L ET AL: "TRANSPLANTATION OF HUMAN HEMATOPOIETIC STEM CELLS INTO RAT BRAIN: ANALYSIS OF CELL SURVIVAL AND DIFFERENTIATION" ABSTRACTS OF THE SOCIETY FOR NEUROSCIENCE, SOCIETY FOR NEUROSCIENCE, WASHINGTON, DC, US, vol. 27, no. 1, 10 November 2001 (2001-11-10), page 632, XP001098867 ISSN: 0190-5295
- FERRERO D ET AL: "MOBILISED PERIPHERAL BLOOD PROGENITOR CELLS GIVE RISE IN VITRO TO CELLS EXPRESSING NEURONAL PHENOTYPE" BLOOD, W.B.SAUNDERS COMPANY, ORLANDO, FL, vol. 98, no. 11, PART 02, 16 December 2001 (2001-12-16), page 1238, XP001062755 ISSN: 0006-4971
- ORTIZ-GONZALEZ XILMA R ET AL: "Neural induction of adult bone marrow and umbilical cord stem cells." CURRENT NEUROVASCULAR RESEARCH JUL 2004, vol. 1, no. 3, July 2004 (2004-07), pages 207-213, XP001538190 ISSN: 1567-2026
- CASTRO RAYMOND F ET AL: "Failure of bone marrow cells to transdifferentiate into neural cells in vivo" SCIENCE (WASHINGTON D C), vol. 297, no. 5585, 23 August 2002 (2002-08-23), page 1299, XP002487072 ISSN: 0036-8075
- WAGERS AMY J ET AL: "Little evidence for developmental plasticity of adult hematopoietic stem cells" SCIENCE (WASHINGTON D C), vol. 297, no. 5590, 27 September 2002 (2002-09-27), pages 2256-2259, XP002487073 ISSN: 0036-8075
- GAZITT YAIR ET AL: "Expression of adhesion molecules on CD34+ cells in peripheral blood of non-Hodgkin's lymphoma patients mobilized with different growth factors" STEM CELLS (MIAMISBURG), vol. 19, no. 2, 2001, pages 134-143, XP002487070 ISSN: 1066-5099
- CAO T M ET AL: "Rapid engraftment after allogeneic transplantation of density-enriched peripheral blood CD34+ cells in patients with advanced hematologic malignancies" CANCER 20010615 US, vol. 91, no. 12, 15 June 2001 (2001-06-15), pages 2205-2213, XP002487071 ISSN: 0008-543X
- GOOLSBY J ET AL: "Hematopoietic progenitors express neural genes" PROCEEDINGS OF THE NATIONAL ACADEMY OF SCIENCES OF USA, NATIONAL ACADEMY OF SCIENCE, WASHINGTON, DC, vol. 100, no. 25, 9 December 2003 (2003-12-09), pages 14926-14931, XP002339337 ISSN: 0027-8424

## Description

### BACKGROUND OF THE INVENTION

Since the discovery of stem cells, it has been understood that they have significant potential to effectively treat many diseases [1]. Pluripotent embryonic stem cells derived from embryos and fetal tissue have the potential to produce more than 200 different known cell types, and thus can potentially replace dying or damaged cells of any specific tissue [2, 3]. Stem cells differ from other types of cells in the body, and, regardless of their source, are capable of dividing and renewing themselves for long periods. In addition, stem cells can give rise to specialized cell types.

Stem cells have been identified in most organs and tissues, and can be found in adult animals and humans. Committed adult stem cells (also referred as somatic stem cells) were identified long ago in bone marrow (BM). Adult stem cells were traditionally thought as having limited self-renewal and differentiation capabilities, restricted to their tissue of origin [1, 4, 5, 6, 7]. These limits are now being challenged by an overwhelming amount of research demonstrating both stem cell plasticity (the ability to differentiate into mature cell types different from their tissue of origin) and therapeutic potential [8, 9,10, 11, 12,13]. For example, recent reports support the view that cells derived from hematopoietic stem cells (HSCs) can differentiate into cells native to the adult brain [12], providing additional evidence for the plasticity of such stem cells.

The HSC is the best characterized stem cell. This cell, which originates in bone marrow, peripheral blood, cord blood, the fetal liver, and the yolk sac, generates blood cells and gives rise to multiple hematopoietic lineages. As early as 1998 researchers reported that pluripotent stem cells from bone marrow can, under certain conditions, develop into several cell types different from known hematopoietic cells [14, 15, 16, 17, 18, 19, 20, 21, 22, 23, 24, 25]. Such an ability to change lineage is referred to as cellular transdifferentiation or cell plasticity.

To date, the general incapacity of the central nervous system (CNS) to regenerate substantially has limited the success of neuroscientists to develop therapies for many traumatic, degenerative, inflammatory, and even severe infectious disorders of the brain. However, the possibility of the eventual use of stem cells in CNS treatments has raised hopes for CNS cell based therapy.

Bone marrow-derived stem cells (BMSCs) have already been shown to have the ability to differentiate into neurons, and other cell types such as adipocytes, chondrocytes, osteocytes, hepatocytes, endothelial cells, and skeletal muscle cells, [26, 27, 28, 29, 30].

The process of stem cell differentiation is controlled by internal signals, which are activated by genes within the cell, and by external signals for cell differentiation that include chemicals secreted by other cells, physical contact with neighboring cells, and certain molecules in the microenvironment [31, 32].

Successful attempts have been made in vitro and in vivo to induce differentiation of adult stem cells into other cells. Several groups have recently found that in vivo BM cells can give rise to astrocytes and oligodendrocytes (both cell types originate from the neuroectoderm) in the murine brain [33, 34, 35]. Indeed even neurons have been found to express markers of the transplanted bone marrow in chimeric mice [35, 36, 37, 38]. In the cerebellum, fully developed Purkinje cells expressing GFP have been reported after transplantation of GFP-marked BM stem cells [38, 39].

Tissue injury may be one of the stimulants for the recruitment of stem cells to an injured site, by causing changes in the tissue environment, thereby drawing stem cells from peripheral blood, as well as triggering tissue replacement by locally-resident stem cells. Reports of elevated levels of chemokines and chemokine receptors such as CXCR4-SDF may explain some of this in vivo stem cell recruitment [40]. Engraftment of bone marrow-derived microglial and astroglial cells is significantly enhanced after CNS injury [41, 42].

Regenerative medicine in general and especially regeneration of CNS damaged tissue is an emerging scientific field with implications for both basic and practical research. Stem and progenitor cells are applied in a form of cellular therapy for local tissue repair and regeneration [43, 44].

The apparent plasticity of BM stem cells has raised hopes for their use in cell-based repair strategies within the CNS. In mouse models of neurological disorders, the transplantation of mesenchymal stem cells (MSC) has resulted in improvement in several studies:
1. Intravenous, intracarotid and intracerebral administration of MSCs after cerebral ischemia improved behavioral recovery in mice and rats [45,46,47,48]. Furthermore, bone marrow-derived cells also contributed to neovascularization after cerebral ischemia in mice [49, 50];
2. In the MPTP (methyl-phenyl-tetrahydropyridine) mouse model of Parkinson's disease, intrastriatal transplantation of MSCs has promoted functional recovery [51];
3. Rats injected with MSCs after spinal contusion and traumatic brain injury showed long-term improvement of locomotor function [52, 53];
4. MSCs were found to remyelinate the rat spinal cord after focal demyelination, and to improve conduction velocity [54].

The following references may be of interest:
1. Leblond C.P. (1964), "Classification of cell populations on the basis of their proliferative behaviour," Natl. Cancer Inst. Monogr. 14:119-150
2. Evans M.J. and Kaufman M.H. (1981), "Establishment in culture of pluripotential cells from mouse embryos," Nature 292:154-156
3. Donovan P.J. and Gearhart J. (2001), "The end of the beginning for pluripotent stem cells," Nature 414:92-97
4. Spradling A. et al. (2001), "Stem cells find their niche," Nature 414:98-104
5. Weissman I.L. et al. (2001), "Stem and progenitor cells: origins, phenotypes, lineage commitments, and transdifferentiations," Annu. Rev. Cell. Dev. Biol. 17:387-403
6. Weissman I.L. (2000), "Stem cells: units of development, units of regeneration, and units in evolution," Cell 100:157-68
7. Cheng A, Wang S, Cai J, Rao MS, Mattson MP (2003), "Nitric oxide acts in a positive feedback loop with BDNF to regulate neural progenitor cell proliferation and differentiation in the mammalian brain," Dev Biol. 258(2):319-33
8. Cousin B, Andre M, Arnaud E, Penicaud L, Casteilla L (2003), "Reconstitution of lethally irradiated mice by cells isolated from adipose tissue," Biochem Biophys Res Commun. 301(4):1016-22
9. Anderson D.J., Gage, F.H., and Weissman, I.L. (2001), "Can stem cells cross lineage boundaries?" Nat. Med. 7:393-395
10. Robey P.G. (2000), "Stem cells near the century mark," J. Clin. Invest. 105:1489-1491
11. Eisenberg LM, Burns L, Eisenberg CA (2003), "Hematopoietic cells from bone marrow have the potential to differentiate into cardiomyocytes in vitro," Anat Rec. 274A(1):870-82
12. Brazelton TR, Rossi FM, Keshet GI, Blau HM (2000), "From marrow to brain: expression of neuronal phenotypes in adult mice," Science 290(5497):1775-9
13. Slack, J.M. (2000), "Stem cells in epithelial tissues," Science 287:1431-1433
14. Jackson KA, Mi T, Goodell MA (1999), "Hematopoietic potential of stem cells isolated from murine skeletal muscle," Proc Natl Acad Sci U S A 96(25): 14482-6
15. Ferrari G., Cusella-De Angelis G., Coletta M., Paolucci E., Stornaiuolo A., Cossu G., and Mavilio F. (1998), "Muscle regeneration by bone marrow-derived myogenic progenitors," Science 279:528-30
16. Lagasse E, Connors H, Al-Dhalimy M, Reitsma M, Dohse M, Osborne L, Wang X, Finegold M, Weissman IL, Grompe M (2000), "Purified hematopoietic stem cells can differentiate into hepatocytes in vivo," Nat Med. 6:1229-34
17. Hirschi, K. K., and Goodell, M. A. (2002), "Hematopoietic, vascular and cardiac fates of bone marrow-derived stem cells," Gene Ther. 9:648-652
18. Theise N.D. et al. (2000), "Liver from bone marrow in humans," Hepatology 32:11-16
19. KJeeberger W. et al. (2002), "High frequency of epithelial chimerism in liver transplants demonstrated by microdissection and STR-analysis," Hepatology 35:110-116
20. Weimann J.M. et al. (2003), "Contribution of transplanted bone marrow cells to Purkinje neurons in human adult brains," Proc. Natl. Acad. Sci. USA 100:2088-2093
21. Quaini F. et al. (2002), "Chimerism of the transplanted heart," N. Engl. Med 346:5-15
22. Blau H.M. et al. (2001), "The evolving concept of a stem cell: entity or function?" Cell 105:829-841
23. Goodell M.A. et al. (2001), "Stem cell plasticity in muscle and bone marrow," Ann. NY Acad. Sci. 938:208-218
24. Krause D.S. (2002), "Plasticity of marrow-derived stem cells," Gene Ther. 9:754-758
25. Wulf G.G. et al. (2001), "Somatic stem cell plasticity," Exp Hematol. 29:1361-1370
26. Pittenger M.F. et al. (1999), "Multilineage potential of adult human mesenchymal stem cells," Science 284:143-147
27. Liechty K.W. et al. (2000), "Human mesenchymal stem cells engraft and demonstrate site-specific differentiation after in utero transplantation in sheep," Nature Med. 6:1282-1286
28. Jang YY, Collector MI, Baylin SB, Diehl AM, Sharkis SJ (2004), "Hematopoietic stem cells convert into liver cells within days without fusion," Nat Cell Biol. 6(6):532-9. Epub 2004 May 09
29. Bittner R.E., Schofer C., Weipoltshammer K., Ivanova S., Streubel B., Hauser E., Freilinger M., Hoger H., Elbe-Burger A., and Wachtler F. (1999), "Recruitment of bone-marrow-derived cells by skeletal and cardiac muscle in adult dystrophic mdx mice," Anat. Embryol. (Berl) 199:391-396
30. Mezey E, Chandross KJ, Harta G, Maki RA, McKercher SR (2000), "Turning blood into brain: cells bearing neuronal antigens generated in vivo from bone marrow," Science. 290(5497):1779-82
31. Douglas W.L., Dimmeler S. (2004), "Therapeutic angiogenesis and vasculogenesis for ischemic diseases. Part I: Angiogenic cytokines," Circulation 109:2487-2491
32. Douglas W.L., Dimmeler S. (2004), "Therapeutic angiogenesis and vasculogenesis for ischemic diseases. Part II: Cell-based therapy," Circulation 109:2692-2697
33. Eglitis MA,Mezey E (1997) Hematopoietic cells differentiate into both microglia and macroglia in the brains of adult mice. Proc Natl Acad Sci U S A 94:4080-4085.
34. Bonilla S, Alarcon P, Villaverde R, Aparicio P, Silva A, Martinez S (2002) Haematopoietic progenitor cells from adult bone marrow differentiate into cells that express oligodendroglial antigens in the neonatal mouse brain. Eur J Neurosci 15:575-582.
35. Corti S, Locatelli F, Strazzer S, Salani S, Del Bo R, Soligo D, Bossolasco P, Bresolin N, Scarlato G, Comi GP (2002) Modulated generation of neuronal cells from bone marrow by expansion and mobilization of circulating stem cells with in vivo cytokine treatment. Exp Neurol 177:443-452.
36. Brazelton TR, Rossi FM, Keshet GI, Blau HM (2000) From marrow to brain: expression of neuronal phenotypes in adult mice. Science 290:1775-1779.
37. Mezey E, Chandross KJ, Harta G, Maki RA, McKercher SR (2000) Turning blood into brain: cells bearing neuronal antigens generated in vivo from bone marrow. Science 290:1779-1782.
38. Priller J, Persons DA, Klett FF, Kempermann G, Kreutzberg GW, Dirnagl U (2001b) Neogenesis of cerebellar Purkinje neurons from gene-marked bone marrow cells in vivo. J Cell Biol 155:733-738.
39. Wagers AJ, Sherwood RI, Christensen JL, Weissman IL (2002) Little evidence for developmental plasticity of adult hematopoietic stem cells. Science 297:2256-2259..
40. Kollet O, Shivtiel S, Chen YQ. et al. (2003), "HGF, SDF-1, and MMP-9 are involved in stress-induced human CD34+ stem cell recruitment to the liver," J Clin Invest. 112(2): 160-9
41. Eglitis MA, Dawson D, Park KW,Mouradian MM (1999) Targeting of marrow-derived astrocytes to the ischemic brain. Neuroreport 10:1289-1292.
42. Priller J, Flogel A, Wehner T, Boentert M, Haas CA, Prinz M, Fernandez-Klett F, Prass K, Bechmann I, de Boer BA, Frotscher M, Kreutzberg GW, Persons DA, Dirnagl U (2001 a) Targeting gene-modified hematopoietic cells to the central nervous system: use of green fluorescent protein uncovers microglial engraftment. Nat Med 7:1356-1361.
43. Bianco, P. and Robey P.G. (2001), "Stem cells in tissue engineering," Nature 414:118-121
44. Lagasse E. et al. (2001), "Toward regenerative medicine," Immunity 14:425-436
45. Li Y, Chopp M, Chen J,Wang L, Gautam SC, Xu YX, Zhang Z (2000) Intrastriatal transplantation of bone marrow nonhematopoietic cells improves functional recovery after stroke in adult mice. J Cereb Blood Flow Metab 20:1311-1319
46. Li Y, Chen J,Wang L, Lu M, Chopp M (2001a) Treatment of stroke in rat with intracarotid administration of marrow stromal cells. Neurology 56:1666-1672
47. Chen J, Li Y, Wang L, Zhang Z, Lu D, Lu M, Chopp M (2001) Therapeutic benefit of intravenous administration of bone marrow stromal cells after cerebral ischemia in rats. Stroke 32:1005-1011.
48. Zhao LR, Duan WM, Reyes M, Keene CD, Verfaillie CM, Low WC (2002) Human bone marrow stem cells exhibit neural phenotypes and ameliorate neurological deficits after grafting into the ischemic brain of rats. Exp Neurol 174:11-20
49. Zhang ZG, Zhang L, Jiang Q, Chopp M (2002) Bone marrow-derived endothelial progenitor cells participate in cerebral neovascularization after focal cerebral ischemia in the adult mouse. Circ Res 90:284-288
50. Hess DC, Hill WD, Martin-Studdard A, Carroll J, Brailer J, Carothers J (2002) Bone marrow as a source of endothelial cells and NeuN-expressing cells after stroke. Stroke 33:1362-1368
51. Li Y, Chen J,Wang L, Zhang L, Lu M, Chopp M (2001b) Intracerebral transplantation of bone marrow stromal cells in a 1-methyl-4-phenyl-1,2,3,6-tetrahydropyridine mouse model of Parkinson's disease. Neurosci Lett 316:67-70
52. Mahmood A, Lu D,Wang L, Li Y, Lu M, Chopp M (2001) Treatment of traumatic brain injury in female rats with intravenous administration of bone marrow stromal cells. Neurosurgery 49:1196-1203
53. Hofstetter CP, Schwarz EJ, Hess D,Widenfalk J, El Manira A, Prockop DJ, Olson L (2002) Marrow stromal cells form guiding strands in the injured spinal cord and promote recovery. Proc Natl Acad Sci U S A 99:2199-2204
54. Akiyama Y, Radtke C, Kocsis JD (2002) Remyelination of the rat spinal cord by transplantation of identified bone marrow stromal cells. J Neurosci 22:6623-6630
55. Hershfinkel M, Moran A, Grossman N et al. A zinc-sensing receptor triggers the release of intracellular Ca2+ and regulates ion transport. Proc Natl Acad Sci USA 2001;98:11749-111754

### SUMMARY OF THE INVENTION

The recited invention provided a method comprising in vitro stimulating a core cell population (CCP) derived from peripheral blood of a donor as described in the claims.

In the context of the present patent application and in the claims, a "core cell population" (CCP) is a population of at least 5 million cells which have a density of less than 1.072 g/ml, and at least 1.5% of which are CD34+CD45-/dim. (That is, at least 75,000 of the cells are both (a) CD34 positive and (b) CD45 negative or CD45 dim.)

For some applications, at least 2% of the 5 million cells are CD34+CD45-/dim. (That is, at least 100,000 of the cells are both (a) CD34 positive and (b) CD45 negative or CD45 dim.)

A method for producing a neural progenitor/precursor cell population (NPCP) is discussed comprising (a) processing cells extracted from a mammalian cell donor to yield a CCP, and (b) stimulating the CCP to differentiate into the neural progenitor/precursor cell population. In the context of the present patent application and in the claims, "progenitor/precursor" cells are partially differentiated cells that are able to divide and give rise to differentiated cells.

While for some applications described herein the density of the cells in the CCP is less than 1.072 g/ml (as described), for some applications, the CCP has at least 5 million cells having a density of less than 1.062 g/ml.

In the context of the present patent application, an "elemental cell population" (ECP) is a population of at least 5 million cells which have a density of less than 1.072 g/ml, at least 1.5% of which are CD34+CD45-/dim, and at least 30% of which are CD 14+.

Typically, but not necessarily, at least 30% or 40% of the cells in the ECP are CD14+. Alternatively or additionally, at least 40%, 50%, 60%, or 70% of the cells are CD31+.

Typically, but not necessarily, at least 2% of the cells in the ECP are CD34+CD45-/dim. For some applications, the ECP has at least 5 million cells having a density of less than 1.062 g/ml It is typically but not necessarily the case that a CCP is also an ECP. It is noted that the procedures discussed herein in relation to a CCP may also be performed in relation to an ECP.

For some applications, the CCP-derived progenitor cells are used as a therapeutic cell product (e.g., for cancer therapy, for tissue regeneration, for tissue engineering, and/or for tissue replacement), as a research tool (e.g., for research of signal transduction, or for screening of growth factors), and/or as a diagnostic tool and/or for gene therapy. When the CCP-derived progenitor cells and/or when CCP-derived partially-differentiated cells are used as a therapeutic cell product, they are typically administered to a patient, in whom the progenitor cells mature into the desired cell types themselves (e.g., neurons, astrocytes, glial cells, oligodendrocytes, photoreceptors, etc.). Alternatively, CCP-derived fully-differentiated cells are used as a therapeutic cell product, and are typically administered to a patient, in whom they can regenerate damaged tissue structure and/or function.

A result of a stage in a process described herein may be used as a diagnostic indicator. For example, pathology of a patient may be indicated if an in vitro procedure performed on extracted blood of the patient does not produce a CCP, when the same procedure would produce a CCP from cells extracted from a healthy volunteer. Alternatively or additionally, a pathology of a patient may be indicated if an in vitro stimulation procedure performed on an autologous CCP does not produce a desired number of a particular class of progenitor cells, when the same procedure would produce the desired number of a particular class of progenitor cells from a CCP derived from cells of a healthy volunteer.

When hematopoietic stem cells are used as source cells to create the CCP, the resultant CCP is typically, but not necessarily, characterized in that at least 30% or 40% of the cells in the CCP are CD 14+, at least 40%, 50%, 60%, or 70% of the cells in the CCP are CD31+, and/or at least 2.2% or at least 2.5% of the cells are CD34+CD45-/dim.

Typically, but not necessarily, the process of stimulating the CCP takes between about 3 and about 15 days, or between about 15 and about 60 days. Alternatively, stimulating the CCP takes less than 3 days, or more than 60 days.

The mammalian cell donor may be human or non-human, as appropriate. For some applications, the mammalian cell donor ultimately receives an administration of a product derived from the CCP, while for other applications, the mammalian cell donor does not receive such a product. Stem cells that can be used to produce the CCP are derived from peripheral blood.

The CCP is typically prepared by generating or obtaining a single cell suspension from this source. For example, mobilized blood mononuclear cells may be extracted using a 1.077g/ml density gradient (e.g., a Ficoll (TM) gradient, including copolymers of sucrose and epichlorohydrin). (It is noted that such a gradient is not used for all applications, e.g., for applications in which a single cell suspension is generated from a non-hematopoietic source such as olfactory bulb, mucosal or skin cells.) The output of this gradient is then typically passed through a second gradient (e.g., a Percoll (TM) gradient, including polyvinylpyrrolidone-coated silica colloids), suitable for selecting cells having a density less than 1.072 g/ml or less than 1.062 g/ml. These selected cells are then typically increased in number, in vitro, until they become a CCP. As appropriate, other density gradients may be used, in addition to or instead of those cited above. For example, an OptiPrep (TM) gradient, including an aqueous solution of Iodixanol, and/or a Nycodenz (TM) gradient may also be used.

The CCP is typically stimulated to generate neural progenitor cells of one or more of the following cell classes:
CNS neurons;
oligodendrocytes;
astrocytes;
peripheral nervous system (PNS) neurons; and
retinal cells (including, but not limited to photoreceptors, pigment epithelium cells, or retinal ganglion cells).

For some applications, the CCP is transfected with a gene prior to the stimulation of the CCP, whereupon the CCP differentiates into a population of desired progenitor cells containing the transfected gene. Typically, these progenitor cells are then administered to a patient. Alternatively or additionally, a gene is transfected into the neural progenitor/precursor cell population for use for gene therapy.

To stimulate the CCP to differentiate into a desired class of progenitor cells, or in association with stimulation of the CCP to differentiate into a desired class of progenitor cells, the CCP is typically directly or indirectly co-cultured with "target tissue" from an organ representing a desired final state of the progenitor cells. For example, the target tissue may include brain or similar tissue when it is desired for the progenitor cells to differentiate into brain tissue. Other examples include.
(a) co-culturing the CCP with peripheral nerves (and/or culturing the CCP in conditioned medium derived therefrom), to induce differentiation of the CCP into peripheral neurons;
(b) co-culturing the CCP with central nervous system (CNS) nerves (and/or culturing the CCP in conditioned medium derived therefrom), to induce differentiation of the CCP into CNS neurons;
(c) co-culturing the CCP with retinal tissue (and/or culturing the CCP in conditioned medium derived therefrom), to induce differentiation of the CCP into retinal tissue. The retinal tissue may include, for example, one or more of: pigment epithelium, or photoreceptors. As appropriate, the retinal tissue may comprise fetal retinal tissue, embryonic retinal tissue, or mature retinal tissue.

Although in some embodiments, co-culturing is performed with sample tissues that are target tissues, as described above, in other embodiments, the CCP is directly or indirectly co-cultured with a sample tissue that does not itself represent a desired final state of the progenitor cells.

For some applications, slices or a homogenate of the target tissue are used for co-culturing, although other techniques for preparing the target tissue will be apparent to a person of ordinary skill in the art who has read the disclosure of the present patent application.

The target tissue may be in essentially direct contact with the CCP, or separated therefrom by a semi-permeable membrane. As appropriate, the target tissue may be autologous, syngeneic, allogeneic, or xenogeneic with respect to the source tissue from which the CCP was produced. Alternatively or additionally, the CCP is cultured in a conditioned medium made using target tissue (e.g., a target tissue described hereinabove), that is autologous, syngeneic, allogeneic, or xenogeneic with respect to the source tissue from which the CCP was produced. For some applications, the target tissue and the CCP are cultured together in the conditioned medium. It is noted that the source of the target tissue may also be tissue from a cadaver, and/or may be lyophilized, fresh, or frozen.

To produce a desired class of progenitor cells, cells from the CCP may be cultured in the presence of stimulation caused by "stimulation factors," e.g., one or more antibodies, cytokines and/or growth factors such as: anti-CD34, anti-CD133, anti-CD117, LIF, EPO, IGF, b-FGF, M-CSF, GM-CSF, TGF alpha, TGF beta, VEGF, BHA, B27, F12, BDNF, GDNF, NGF, NT3, NT4/5, S-100, CNTF, EGF, NGF3, CFN, ADMIF, estrogen, prolactin, an adrenocorticoid, glutamate, serotonin, acetylcholine, NO, retinoic acid (RA), heparin, insulin, forskolin, and/or cortisone, and/or a derivative of one of these. It is to be appreciated that the particular stimulation factors described herein are by way of illustration and not limitation. As appropriate, these may be utilized in a concentration of between about 100 pg/ml and about 100 µg/ml (or molar equivalents). In some cases, medium additives are added at volume ratios of about 1:1 to about 1:30, or about 1:30 to about 1:500 from the total volume of the medium. Typically, particular stimulation factors are selected in accordance with the particular class of progenitor cells desired (e.g., to induce neural progenitor cells, one or more of the following stimulation factors or media additives are used: BHA, BDNF, NGF, NT3, NT4/5, EGF, NGF3, S-100, CNTF, GDNF, CFN, ADMIF, B27, F12 and acetylcholine).

For some applications, the stimulation factors are introduced to the CCP in a soluble form, and/or in an aggregated form, and/or attached to a surface of a culture dish. In an embodiment, the CCP is incubated on a surface comprising a growth-enhancing molecule other than collagen or fibronectin. The growth-enhancing molecule may comprise, for example, BDNF or another suitable antibody or factor described herein. As appropriate, the growth-enhancing molecule may be mixed with collagen or fibronectin, or may be coated on the surface in a layer separate from a layer on the surface that comprises collagen or fibronectin. Alternatively, the only growth-enhancing molecule(s) on the surface is collagen and/or fibronectin and/or autologous plasma.

Following stimulation of the CCP, the resultant product is typically tested to verify that it has differentiated into a desired form. For example, when neural progenitor cells are the desired product, the product typically comprises one or more of: CD34, CD117, CD44, Neu-N, nestin, microtubule associated protein-1 (MAP-1), MAP Tau, microtubule associated protein-2 (MAP-2), neurofilament NF200, neuron-specific enolase (NSE), choline acetyltransferase (ChAT) neuronal class III β-Tubulin, glutamic acid decarboxylase (GAD), glutamic acid, gamma-aminobutyric acid (GABA), oligodendrocyte marker (04), myelin basic protein (MBP), galactocerebroside (GalC), glial fibrillary acidic protein (GFAP), CD211b, dopamine, norepinephrine, epinephrine, glycine, glutamate, acetylcholine, serotonin, and endorphin. The supernatant may also include one or more of the above, or neurotrophins, such as S-100, GDNF, CNTF, BDNF, NGF, NT3, NT4/5, which may be secreted by the NPCP.

Typically, greater than 1.5% of the cell population demonstrates one or more of these molecules. Alternatively or additionally, neural progenitor cells are typically positive for one or more of: Nestin, NSE, Notch, numb, Musasbi-1, presenilin, FGFR4, Fz9, SOX 2, GD2, rhodopsin, recoverin, calretinin, PAX6, RX and Chx10.

For some applications, an effort is made to minimize the time elapsed from collection of cells from the cell donor until the CCP-derived progenitor cells are used (e.g., for administration into a patient). Alternatively, cells are preserved at one or more points in the process. For example, the CCP may be frozen prior to the stimulation thereof that generates progenitor cells. In another example, the CCP are stimulated in order to generate desired progenitor cells, and these progenitor cells are frozen. In either of these cases, the frozen cells may be stored and/or transported, for subsequent thawing and use.

By way of illustration and not limitation, it is noted that certain applications are suitable for large-scale commercialization, including freezing and transport, such as (a) generation of stores of CCPs, (b) generation of stores of NPCPs, and (c) stem cell banks where individuals may store a CCP or differentiated NPCP cells, for possible later use. "Transport," in this context, means transport to a remote site, e.g., a site greater than 10 km or 100 km away from a site where the CCP is first created.

For some applications, the CCP is cultured for a period lasting between about 1 and about 60 days in a culture medium without serum (serum free) or in a culture medium comprising less than about 5% serum. Alternatively, the CCP is cultured for a period lasting between about 1 and about 60 days in a culture medium comprising greater than about 10% serum. One of these periods may follow the other of these periods.

For some applications, the CCP is cultured, in serum-free or low-serum conditions for a certain period, in a culture medium comprising less than about 10% serum (e.g., less than 1% or 0.01% serum, or being serum free), and, in high-serum conditions for another period, in a culture medium comprising greater than or equal to about 10% serum. Culturing the CCP during the serum-free or low-serum period may comprise culturing the CCP for a duration of between about 1 and about 60 days. Alternatively or additionally, culturing the CCP during the high-serum time period comprises culturing the CCP for a duration of between about 1 and about 60 days. Typically, culturing the CCP during the serum-free or low-serum period is performed prior to culturing the CCP during the higb-serum time period. Alternatively, culturing the CCP during the low-serum time period is performed following culturing the CCP during the high-serum time period.

For some applications, the CCP is cultured in the presence of one or more proliferation-differentiation-enhancing agents, such as anti-CD34, anti-CD133, anti-CD117, LIF, EPO, IGF, b-FGF, M-CSF, GM-CSF, TGF alpha, TGF beta, VEGF, BHA, B27, F12, BDNF, NGF, NT3, NT4/5, S-100, CNTF, GDNF, EGF, NGF3, CFN, ADMIF, estrogen, prolactin, an adrenocorticoid, glutamate, serotonin, acetylcholine, NO, retinoic acid (RA), heparin, insulin, forskolin, and/or cortisone, and/or a derivative of any of these.

Techniques described herein may be practiced in combination with (a) techniques described in one or more of the references cited herein, techniques described in US2008/0220466 and/or WO 05/120090, and/or (c) techniques described in WO 06/064501.

A method is provided comprising culturing the CCP in a first container during a first portion of a culturing period; removing at least some cells of the CCP from the first container at the end of the first portion of the period; and culturing, in a second container during a second portion of the period, the cells removed from the first container. For example, removing at least some of the CCP cells may comprise selecting for removal cells that adhere to a surface of the first container.

If cells from a progenitor/precursor cell population derived from a CCP are to be transplanted into a human, they should be generally free from any bacterial or viral contamination. In addition, in the case of a NPCP the following conditions should typically be met:
(I) Cells should be morphologically characterized as (a) larger in size than lymphocytes, and/or (b) having irregular perikarya, from which filamentous or tubular extensions spread, contacting neighboring cells and forming net-like organizations, and/or (c) granulated or dark nucleated.
(II) Final cell suspension should generally contain at least 1 million cells expressing one or more of the following: CD34, CD117, CD44, Neu-N, nestin, microtubule associated protein-1 (MAP-1), MAP Tau, microtubule associated protein-2 (MAP-2), neurofilament NF200, neuron-specific enolase (NSE), choline acetyltransferase (ChAT) neuronal class III β-Tubulin, glutamic acid decarboxylase (GAD), glutamic acid, gamma-aminobutyric acid (GABA), oligodendrocyte marker (04), myelin basic protein (MBP), galactocerebroside (GalC), glial fibrillary acidic protein (GFAP), CD211b, dopamine, norepinephrine, epinephrine, glutamate, glycine, acetylcholine, serotonin, and endorphin. The supernatant may also include
   one or more of the above, or neutrophins, such as S-100, GDNF, CNTF, BDNF, NGF, NT3, NT4/5, which may be secreted by the NPCP.

It is noted that the cells in CCPs generated from various tissues typically can be characterized as having greater than 80% viability.

It is noted that the CPPS generated from blood, bone marrow, and umbilical cord blood, typically have greater than 70% of their cells being CD45+.

The present invention provides a method including in vitro stimulating a core cell population (CCP) of at least 5 million cells that have a density of less than 1.072 g/ml, and at least 1.5% of which are CD34+CD45-/dim, to differentiate into a neural progenitor/precursor cell population (NPCP), wherein at least some of the cells of the neural progenitor/precursor cell population express Neu-N, at least some of the cells of the neural progenitor/precursor cell population express nestin, at least some of the cells of the neural progenitor/precursor cell population express beta-tubulin, at least some of the cells of the neural progenitor/precursor cell population express 04, and at least some of the cells of the neural progenitor/precursor cell population express GFAP.

In an embodiment, the CCP includes at least 5 million cells that have a density of less than 1.062 g/ml, at least 2% of which are CD34+CD45-/dim, and wherein stimulating the CCP includes stimulating the CCP that has the at least 5 million cells that have a density of less than 1.062 g/ml.

In an embodiment, at least 1.5 million of the cells in the NPCP and at least 1.5% of the cells in the NPCP include at least one molecule or molecular structure selected from the list consisting of: CD34, CD117, CD44, Neu-N, nestin, microtubule associated protein-1 (MAP-1), MAP Tau, microtubule associated protein-2 (MAP-2), neurofilament NF200, neuron-specific enolase (NSE), choline acetyltransferase (ChAT) neuronal class III β-Tubulin, glutamic acid, gamma-aminobutyric acid (GABA), oligodendrocyte marker (04), myelin basic protein (MBP), galactocerebroside (GalC), glial fibrillary acidic protein (GFAP), CD211b, dopamine, norepinephrine, epinephrine, glutamate, glycine, acetylcholine, serotonin, and endorphin.

In an embodiment, the method includes preparing the NPCP as a research tool.

Stimulating the CCP may include only stimulating CCP if the CCP is derived from a mammalian donor.

In an embodiment, the method includes applying cells extracted from a mammalian donor to one or more gradients suitable for selecting cells having a density less than 1.072 g/ml.

In an embodiment, the CCP is characterized by at least 2% of the CCP being CD34+CD45-/dim.

In an embodiment, the CCP is characterized by at least 2.5% of the CCP being CD34+CD45-/dim.

In an embodiment, the CCP is characterized by at least 50% of the CCP being CD14+.

In an embodiment, the CCP is characterized by at least 30% of the CCP being CD14+.

In an embodiment, the CCP is characterized by at least 70% of the CCP being CD31+.

In an embodiment, the CCP is characterized by at least 40% of the CCP being CD31+.

In an embodiment, stimulating the CCP includes stimulating the CCP to differentiate into a pre-designated, desired class of neural progenitor cells.

In an embodiment, stimulating the CCP includes culturing the CCP during a period of between 3 and 60 days in vitro.

The CCP is derived from peripheral blood.

In an embodiment, the method includes deriving the CCP from at least one source selected from the list consisting of: fresh tissue and frozen tissue.

Also disclosed herein is a method that includes identifying an intended recipient of the NPCP, and deriving the CCP from at least one source selected from the list consisting of: tissue autologous to tissue of the intended recipient, tissue syngeneic to tissue of the intended recipient, tissue allogeneic to tissue of the intended recipient, and tissue xenogeneic to tissue of the intended recipient.

In an embodiment, stimulating the CCP includes incubating the CCP in a container having a surface including at least one of: an antibody and autologous plasma.

In an embodiment, stimulating the CCP includes culturing the CCP for a period lasting between 1 and 5 days in a culture medium including less than 0.01% serum.

In an embodiment, stimulating the CCP includes culturing the CCP for a period lasting between 1 and 5 days in a culture medium including less than 5% serum.

In an embodiment, stimulating the CCP includes culturing the CCP for a period lasting between 1 and 5 days in a culture medium including at least 10% serum.

In an embodiment, stimulating the CCP includes culturing the CCP in the presence of at least one of the following: B27, F12, a proliferation-differentiation-enhancing agent, anti-CD34, anti-CD117, LIF, IGF, b-FGF, M-CSF, GM-CSF, TGF alpha, TGF beta, BHA, BDNF, NGF, NT3, NT4/5, S-100, GDNF, CNTF, EGF, NGF3, CFN, ADMIF, estrogen, prolactin, an adrenocorticoid, glutamate, serotonin, acetylcholine, retinoic acid (RA), heparin, insulin, forskolin, cortisone, and a derivative of any of these.

Also disclosed herein is a method that includes preparing the CCP, and facilitating a diagnosis responsive to a characteristic of the preparation of the CCP.

In an embodiment, the method includes freezing the CCP prior to stimulating the CCP.

In an embodiment, the method includes freezing the NPCP.

In an embodiment, the method includes transporting the CCP to a site at least 10 km from a site where the CCP is first created, and stimulating the CCP at the remote site.

In an embodiment, the method includes transporting the NPCP to a site at least 10 km from a site where the NPCP is first created.

The method may include transfecting a gene into the NPCP, and subsequently assessing a level of expression of the gene.

The method may include transfecting a gene into the CCP, and subsequently assessing a level of expression of the gene.

The method may include transfecting into the NPCP a gene identified as suitable for gene therapy.

The method may include transfecting a gene into the CCP prior to stimulating the CCP.

Transfecting the gene may include transfecting into the CCP a gene identified as suitable for gene therapy.

The method may include preparing, as a product for administration to a patient, the NPCP generated by differentiation of the CCP into which the gene has been transfected.

In an embodiment, the method includes preparing the NPCP as a product for administration to a patient.

The patient may have a condition selected from the list consisting of: a neural consequence of a transient ischemic attack (TIA), a neural consequence of ischemic stroke, a circulatory disease, hypertensive neuropathy, venous thrombosis, a cerebrovascular disorder, a cerebrovascular disorder caused by bleeding, a neural consequence of intracerebral hemorrhage, a neural consequence of subdural hemorrhage, a neural consequence of epidural hemorrhage, a neural consequence of subarachnoid hemorrhage, a neural consequence of an arteriovenous malformation, Alzheimer's-related dementia, non-Alzbeimer's-related dementia, vascular dementia, AIDS dementia, hereditary degeneration, Batten's syndrome, traumatic CNS injury, a neural consequence of a CNS neoplasm, a neural consequence of peripheral nervous system surgery, a neural consequence of central nervous system surgery, a radiation injury to the CNS, a neural consequence of a CNS infection, Parkinson's disease, a choreoathetoid syndrome, a progressive supranuclear palsy, spinocerebellar degeneration, multiple sclerosis, a demyelinating diseases, acute disseminated encephalomyelitis, adrenoleukodystrophy, neuromyelitis optica, a neural deficit due to cerebral palsy, a neurological consequence of hydrocephalus, Leber's optic atrophy, a mitochondrial disease, myoclonus epilepsy associated with ragged-end fiber disease (MERRF), mitochondrial encephalomyopathy, a motor neuron disease, progressive muscular atrophy, amyotrophic lateral sclerosis, a cranial nerve lesion, Horner's syndrome, internuclear ophthalmoplegia, Parinaud's syndrome, a gaze palsy, a neural consequence of intoxication, a neural consequence of poisoning, acquired retinal degeneration, age related macular degeneration (AMD), myopic retinopathy, Best's disease, central serous choroidoretinopathy, a vascular retinopathy, diabetic retinopathy, hypertensive retinopathy, glaucoma, retinitis pigmentosa, a hereditary retinopathy, a retinal hole, an ophthalmic mechanical injury affecting a retina, a radiation injury affecting the retina, a retinal vascular occlusion, a retinal deficit caused by retinal detachment, an inner-ear disease, peripheral nervous system degeneration, peripheral nervous system injury, and a peripheral nervous system vascular lesion.

In an embodiment, the method includes facilitating a diagnosis responsive to stimulating the CCP to differentiate into the NPCP.

Facilitating the diagnosis may include assessing an extent to which the stimulation of the CCP produces a particular characteristic of the NPCP.

In an embodiment, stimulating the CCP includes incubating the CCP in a container with a surface including a growth-enhancing molecule other than collagen or fibronectin.

In an embodiment, incubating the CCP further comprises

applying to the surface a layer that includes the growth-enhancing molecule and a separate layer that includes the at least one of: collagen, fibronectin, and autologous plasma.

Stimulating the CCP may include :
during a low-serum time period, culturing the CCP in a culture medium including less than 10% serum; and
during a high-serum time period, culturing the CCP in a culture medium including greater than or equal to 10% serum

Culturing the CCP in the culture medium including less than 10% serum may include culturing the CCP in a culture medium including less than 0.01% serum.

Culturing the CCP during the low-serum time period may include culturing the CCP for a duration of between 1 and 5 days.

Culturing the CCP during the high-serum time period may include culturing the CCP for a duration of between 1 and 30 days.

Culturing the CCP during the low-serum time period may be performed prior to culturing the CCP during the high-serum time period.

Culturing the CCP during the low-serum time period may be performed following culturing the CCP during the high-serum time period.

In an embodiment, stimulating the CCP includes:
culturing the CCP in a first container during a first portion of a culturing period;
removing at least some cells of the CCP from the first container at the end of the first portion of the period; and
culturing, in a second container during a second portion of the period, the cells removed from the first container.

In an embodiment, removing at least some cells of the CCP includes selecting for removal cells that adhere to a surface of the first container.

In an embodiment, removing at least some cells of the CCP includes selecting for removal cells that do not adhere to a surface of the first container.

In an embodiment, the first container includes on a surface thereof a growth-enhancing molecule, and wherein culturing the CCP in the first container includes culturing the CCP in the first container that includes the growth-enhancing molecule.

In an embodiment, the growth-enhancing molecule is selected from the list consisting of: collagen, fibronectin, autologous plasma, a growth factor, and an antibody to a stem cell surface receptor.

In an embodiment, the second container includes on a surface thereof a growth-enhancing molecule, and wherein culturing the CCP in the second container includes culturing the CCP in the second container that includes the growth-enhancing molecule.

In an embodiment, the growth-enhancing molecule is selected from the list consisting of: collagen, fibronectin, autologous plasma, a growth factor, and an antibody to a stem cell surface receptor.

In an embodiment, stimulating includes culturing the CCP with at least one factor derived from a target tissue.

In an embodiment, the method includes preparing a conditioned medium for culturing the CCP therein, the conditioned medium including the factor, the factor being derived from a tissue selected from the list consisting of: peripheral nerve tissue, central nervous system (CNS) tissue, retinal tissue, pigment epithelial tissue, photoreceptor tissue, fetal retinal tissue, embryonic retinal tissue, and mature retinal tissue.

In an embodiment, stimulating includes co-culturing the CCP with a target tissue.

In an embodiment, co-culturing includes preparing the target tissue by a method selected from the list consisting of: slicing the target tissue, homogenizing the target tissue, freezing the target tissue, processing the target tissue by ultrasound, and processing the target tissue by non-ultrasound radiation.

In an embodiment, co-culturing includes:
utilizing the target tissue to produce a conditioned medium; and
co-culturing the CCP with the target tissue in the conditioned medium.

In an embodiment, co-culturing includes separating the target tissue from the CCP by a semi-permeable membrane.

In an embodiment, the method includes designating the target tissue to include a tissue selected from the list consisting of: peripheral nerve tissue, central nervous system (CNS) tissue, retinal tissue, pigment epithelial tissue, photoreceptor tissue, fetal retinal tissue, embryonic retinal tissue, and mature retinal tissue.

There is also provided a method including in vitro stimulating an elemental cell population (ECP) of at least 5 million cells that have a density of less than 1.072 g/ml, at least 1.5% of which are CD34+CD45-/dim, at least 30% of which are CD14+, and/or at least 40% of which are CD31+, to differentiate into a progenitor/precursor cell population (PCP).

For some applications, the ECP includes at least 5 million cells that have a density of less than 1.062 g/ml, at least 2% of which are CD34+CD45-/dim, and stimulating the ECP includes stimulating the ECP that has the at least 5 million cells that have a density of less than 1.062 g/ml.

For some applications, stimulating the ECP includes only stimulating the ECP if the ECP is derived from a mammalian donor. For some applications, the method includes applying cells extracted from a mammalian donor to one or more gradients suitable for selecting cells having a density less than 1.072 g/ml, and deriving the ECP responsive to applying the cells to the gradient.

For some applications, the ECP is characterized by at least 2.5% of the ECP being CD34+CD45-/dim, and stimulating the ECP includes stimulating the ECP having the at least 2.5% of the ECP that are CD34+CD45-/dim. For some applications, the ECP is characterized by at least 50% of the ECP being CD14+, and stimulating the ECP includes stimulating the ECP having the at least 50% of the ECP that are CD14+. For some applications, the ECP is characterized by at least 30% of the ECP being CD14+, and stimulating the ECP includes stimulating the ECP having the at least 30% of the ECP that are CD14+. For some applications, the ECP is characterized by at least 40% or at least 70% of the ECP being CD31+, and stimulating the ECP includes stimulating the ECP having the at least 40% or at least 70% of the ECP that are CD31+.

For some applications, stimulating the ECP includes stimulating the ECP to differentiate into a pre-designated, desired class of progenitor cells.

For some applications, stimulating the ECP includes culturing the ECP during a period of between 3 and 60 days in vitro.

For some applications, the method includes deriving the ECP from at least one source selected from the list consisting of: umbilical cord blood, umbilical cord tissue, neonatal tissue, adult tissue, fat tissue, nervous tissue, bone marrow, mobilized blood, peripheral blood, peripheral blood mononuclear cells, and skin cells. Alternatively, the method includes deriving the ECP from at least one source selected from the list consisting of: fresh tissue and frozen tissue. For some applications, the method includes identifying an intended recipient of the PCP, and deriving the ECP from at least one source selected from the list consisting of: tissue autologous to tissue of the intended recipient, tissue syngeneic to tissue of the intended recipient, tissue allogeneic to tissue of the intended recipient, and tissue xenogeneic to tissue of the intended recipient.

For some applications, stimulating the ECP includes incubating the ECP in a container having a surface including an antibody.

For some applications, stimulating the ECP includes culturing the ECP for a period lasting between 1 and 60 days in a culture medium including serum-free or less than 5% serum. For some applications, stimulating the ECP includes culturing the ECP for a period lasting between 1 and 60 days in a culture medium including at least 10% serum.

For some applications, stimulating the ECP includes culturing the ECP in the presence of at least one of the following: a proliferation-differentiation-enhancing agent, anti-CD34, anti-Tie-2, anti-CD133, anti-CD117, LIF, EPO, IGF, b-FGF, M-CSF, GM-CSF, TGF alpha, TGF beta, VEGF, BHA, B27, F12, BDNF, NGF, NT3, NT4/5, S-100, GDNF, CNTF, EGF, NGF3, CFN, ADMIF, estrogen, prolactin, an adrenocorticoid, glutamate, serotonin, acetylcholine, NO, retinoic acid (RA), heparin, insulin, forskolin, and/or cortisone, and/or a derivative of any of these.

For some applications, the method includes preparing the ECP, and facilitating a diagnosis responsive to a characteristic of the preparation of the ECP.

For some applications, the method includes freezing the ECP prior to stimulating the ECP. For some applications, the method includes freezing the NPCP.

For some applications, the method includes transporting the ECP to a site at least 10 km from a site where the ECP is first created, and stimulating the ECP at the remote site. For some applications, the method includes transporting the NPCP to a site at least 10 km from a site where the NPCP is first created.

The method may include facilitating a diagnosis responsive to stimulating the ECP to differentiate into the NPCP. For some applications, facilitating the diagnosis includes assessing an extent to which the stimulation of the ECP produces a particular characteristic of the NPCP.

The method may include transfecting a gene into the ECP prior to stimulating the ECP. For some applications, the method includes preparing, as a product for administration to a patient, the NPCP generated by differentiation of the ECP into which the gene has been transfected.

Stimulating the ECP may include incubating the ECP in a container with a surface including a growth-enhancing molecule or substance other than collagen or fibronectin or autologous plasma. For some applications, incubating the ECP cells includes incubating the ECP in a container having a surface that includes, in addition to the growth-enhancing molecule, at least one of: collagen, fibronectin, and autologous plasma. For some applications, the method includes mixing the growth-enhancing molecule with the at least one of: collagen and fibronectin and autologous plasma. For some applications, the method includes applying to the surface a layer that includes the growth-enhancing molecule and a separate layer that includes the at least one of: collagen, fibronectin, and autologous plasma.

Stimulating the ECP may include:
during a serum-free or low-serum time period, culturing the ECP in a culture medium including less than 10% serum; and during a high-serum time period, culturing the ECP in a culture medium including greater than or equal to 10% serum.

For some applications, culturing the ECP during the serum-free or low-serum time period includes culturing-the ECP for a duration of between 1 and 60 days. For some applications, culturing the ECP during the high-serum time period includes culturing the ECP for a duration of between 1 and 60 days. For some applications, culturing the ECP during the serum-free or low-serum time period is performed prior to culturing the ECP during the high-serum time period. For some applications, culturing the ECP during the serum-free or low-serum time period is performed following culturing the ECP during the high-serum time period.

Stimulating the ECP may include:
culturing the ECP in a first container during a first portion of a culturing period;
removing at least some cells of the ECP from the first container at the end of the first portion of the period; and
culturing, in a second container during a second portion of the period, the cells removed from the first container.

For some applications, removing at least some cells of the ECP includes selecting for removal cells that adhere to a surface of the first container. For some applications, removing at least some cells of the ECP includes selecting for removal cells that do not adhere to a surface of the first container.

For some applications, the first container includes on a surface thereof a growth-enhancing molecule, and culturing the ECP in the first container includes culturing the ECP in the first container that includes the growth-enhancing molecule.

For some applications, the second container includes on a surface thereof a growth-enhancing molecule, and culturing the ECP in the second container includes culturing the ECP in the second container that includes the growth-enhancing molecule.

Stimulating may include culturing the ECP with at least one factor derived from a target issue. For some applications, the method includes preparing a conditioned medium for culturing the ECP therein, the conditioned medium including the factor, the factor being derived from a tissue selected from the list consisting of: peripheral nerve tissue, central nervous system (CNS) tissue, retinal tissue, pigment epithelial tissue, photoreceptor tissue, fetal retinal tissue and embryonic retinal tissue, mature retinal tissue.

Stimulating may include co-culturing the ECP with a target tissue. For some applications, co-culturing includes freezing the target tissue and/or processing the target tissue by ultrasound or other type of radiation. For some applications, co-culturing includes preparing the target tissue by a method selected from the list consisting of: slicing the target tissue, and homogenizing the target tissue. For some applications, co-culturing including utilizing the target tissue to produce a conditioned medium, and co-culturing the ECP with the target tissue in the conditioned medium. For some applications co-culturing includes separating the target tissue from the ECP by a semi-permeable membrane.

The present invention provides a method including in vitro stimulating a core cell population (CCP) of at least 5 million cells that have a density of less than 1.072 g/ml, and at least 1.5% of which are CD34+CD45-/dim, to differentiate into a neural progenitor/precursor cell population (NPCP), wherein at least some of the cells of the neural progenitor/precursor cell population express Neu-N, at least some of the cells of the neural progenitor/precursor cell population express nestin, at least some of the cells of the neural progenitor/precursor cell population express beta-tubulin, at least some of the cells of the neural progenitor/precursor cell population express 04, and at least some of the cells of the neural progenitor/precursor cell population express GFAP.

In an embodiment, the CCP includes at least 5 million cells that have a density of less than 1.062 g/ml, at least 2% of which are CD34+CD45-/dim, and stimulating the CCP includes stimulating the CCP that has the at least 5 million cells that have a density of less than 1.062 g/ml.

In an embodiment, at least 1.5 million of the cells in the NPCP and at least 1.5% of the cells in the NPCP include at least one molecule or molecular structure selected from the list consisting of: CD34, CD117, CD44, Neu-N, nestin, microtubule associated protein-1 (MAP-1), MAP Tau, microtubule associated protein-2 (MAP-2), neurofilament NF200, neuron-specific enolase (NSE), choline acetyltransferase (ChAT) neuronal class III β-Tubulin, glutamic acid decarboxylase (GAD), glutamic acid, gamma-aminobutyric acid (GABA), oligodendrocyte marker (04), myelin basic protein (MBP), galactocerebroside (GalC), glial fibrillary acidic protein (GFAP), CD211b, dopamine, norepinephrine, epinephrine, glutamate, glycine, acetylcholine, serotonin, and endorphin. The supernatant may also include one or more of the above, or neurotrophins, such as S-100, GDNF, CNTF, BDNF, NGF, NT3, NT4/5, which may be secreted by the NPCP.

In an embodiment, the method includes preparing the NPCP as a research tool.

In an embodiment, stimulating the CCP includes only stimulating the CCP if the CCP is derived from a mammalian donor.

In an embodiment, the method includes applying cells extracted from a mammalian donor to one or more gradients suitable for selecting cells having a density less than 1.072 g/ml.

In an embodiment, the CCP is characterized by at least 2% of the CCP being CD34+CD45-/dim.

In an embodiment, the CCP is characterized by at least 2.5% of the CCP being CD34+CD45-/dim.

In an embodiment, the CCP is characterized by at least 50% of the CCP being CD14+.

In an embodiment, the CCP is characterized by at least 30% of the CCP being CD14+.

In an embodiment, the CCP is characterized by at least 70% of the CCP being CD31+.

In an embodiment, the CCP is characterized by at least 40% of the CCP being CD31+.

In an embodiment, stimulating the CCP includes stimulating the CCP to differentiate into a pre-designated, desired class of neural progenitor cells.

In an embodiment, stimulating the CCP includes culturing the CCP during a period of between 3 and 60 days in vitro.

The present invention will be more fully understood from the following detailed description of embodiments thereof, taken together with the drawings, in which:

### BRIEF DESCRIPTION OF THE DRAWINGS

Figs. 1A-F are photographs of cells, taken at days 4, 8, 12, 15, 19, and 20, respectively, showing morphological changes during the generation of a CCP-derived NPCP, in accordance with an embodiment of the present invention;
Fig. 2 is a photograph showing the morphology of a CCP-derived NPCP cell cluster, in accordance with an embodiment of the present invention;
Figs. 3A-E are photographs showing immunostaining results for a CCP-derived NPCP, in accordance with an embodiment of the present invention;
Fig. 4 is a graph showing the percentage of cells testing positive for the neural markers Nestin and β-Tubulin, in accordance with an embodiment of the present invention;
Fig. 5 is a graph showing calcium levels generated by CCP-derived NPCP cells in response to stimulation by the neurotransmitters glutamate and GABA, in accordance with an embodiment of the present invention; and
Figs. 6A-B are photographs showing a section of a rat's eye, 20 days after implantation of human-CCP-derive NPCP cells, in accordance with an embodiment of the present invention.

### DETAILED DESCRIPTION OF EMBODIMENTS

### Example 1. CCP Characterization before incubation

Ten experiments were carried out in accordance with an embodiment of the present invention, and results are shown in Table 1 below. Peripheral blood was extracted from human volunteers for use in ten respective experiments. In each experiment, a Ficoll gradient was used to generate a population of peripheral blood mononuclear cells (PBMCs). A CCP was generated in accordance with protocols described herein by using a Percoll gradient, further enriching the cell population. Results in Table 1 show the percentages of CD14⁺ and CD34+CD45-/dim cells in the CCP cells, as well as their viability.

**Table 1**

| **Exp.No** | **%Viability** | **%CD45⁺** | **%CD14⁺** | **%CD34⁺CD45^{-/dim}** |
|---|---|---|---|---|
| 1 | 97.86 | 93.46 | 79.98 | 4.07 |
| 2 | 97.61 | 87.10 | 57.14 | 3.48 |
| 3 | 100 | 96.44 | 61.57 | 2.31 |
| 4 | 98.18 | 92.77 | 65.52 | 2.69 |
| 5 | 98.53 | 84.30 | 62.82 | 2.77 |
| 6 | 98.33 | 76.16 | 40.99 | 2.37 |
| 7 | 97.78 | 94.46 | 62.48 | 3.7 |
| 8 | 97.93 | 92.39 | 50.24 | 6.14 |
| 9 | 97.64 | 96.55 | 57.02 | 2.24 |
| 10 | 99.37 | 98.44 | 66.30 | 1.67 |
| **Average** | **98.32** | **91.41** | **60.41** | **3.14** |

### Example 2. CCP Characterization before incubation

Seven independent experiments were carried out in accordance with an embodiment of the present invention, and results are shown in Table 2 below. Peripheral blood was extracted from human volunteers for use in ten respective experiments. In each experiment, a Ficoll gradient was used to generate a population of peripheral blood mononuclear cells (PBMCs). A CCP was generated in accordance with protocols described herein by using a Percoll gradient, further enriching the cell population. Results in Table 2 show the percentages of CD31⁺ in the CCP cells.

**Table 2**

| **Exp No** | **%CD31+** |
|---|---|
| 1 | 87.28 |
| 2 | 85.25 |
| 3 | 82.22 |
| 4 | 81.91 |
| 5 | 75.77 |
| 6 | 86.8415 |
| 7 | 90.90 |
| **Average** | **84.31** |

### Example 3. Morphological follow-up of NPCP generated in the presence of growth factors.

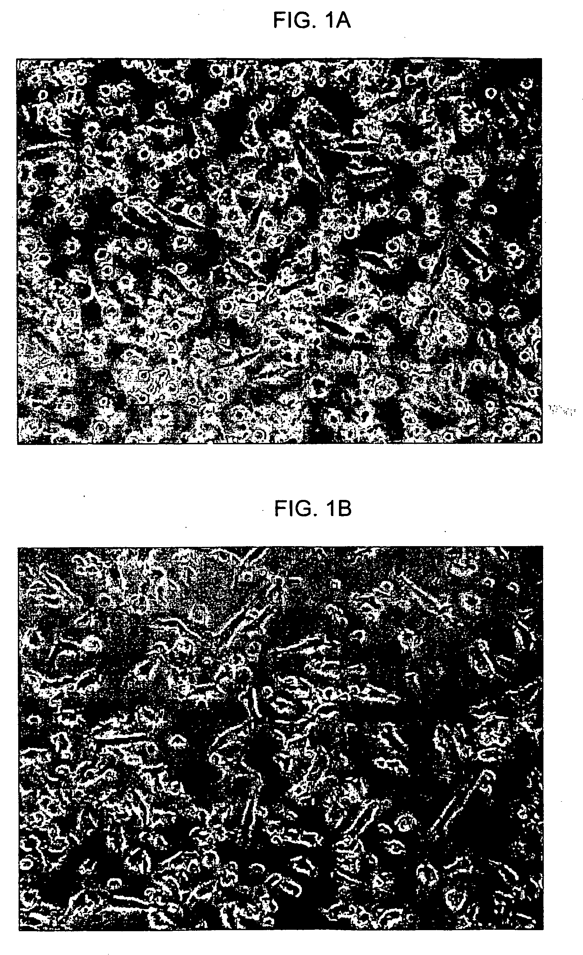

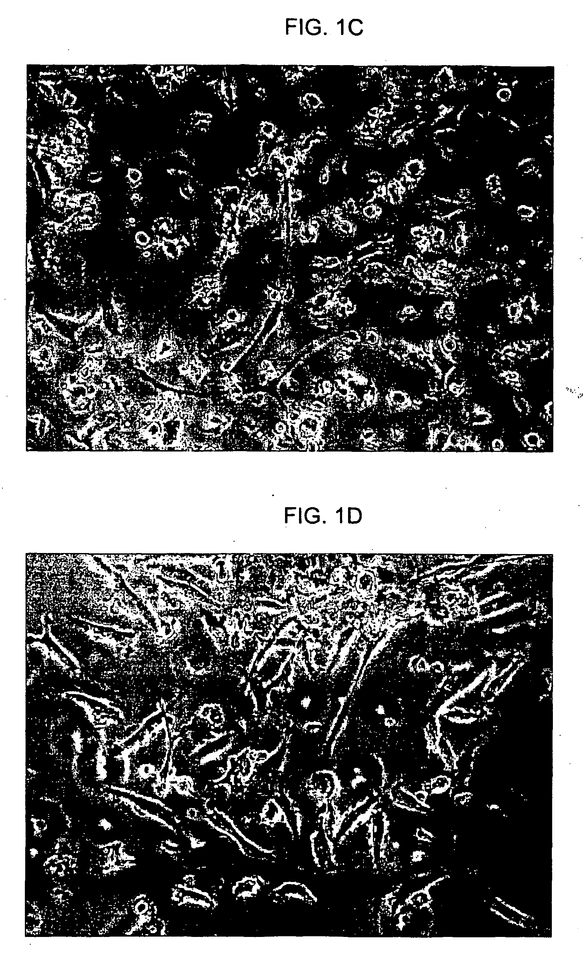

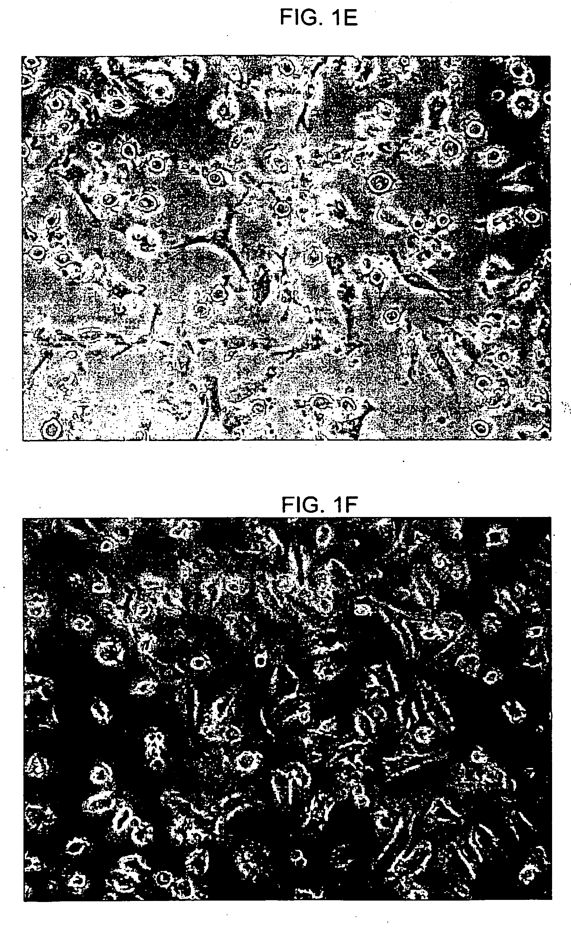

In a separate set of experiments, a morphological follow-up *of in vitro* generation of NPCP was carried out. *The in vitro* generation of the NPCP was carried out in accordance with an embodiment of the present invention, and results are shown in Figure 1.

Figs. 1A-F are photographs of cells, taken at days 4, 8, 12, 15, 19, and 20, respectively, showing morphological changes during the generation of a CCP-derived NPCP, in accordance with an embodiment of the present invention. Nine separate experiments were performed, each experiment using peripheral blood from a separate donor. In each experiment, a Ficoll gradient was used to generate a population of PBMCs. A CCP was generated in accordance with protocols described herein by using a Percoll gradient.

Images in Figure 1 show the morphological changes during the generation of CCP-derived NPCP from one representative experiment. Typically, circle-shaped cells and elongated cells are observed during the first days of the culture (Figure 1A). Subsequently, (Figure 1B-E) the cells increase in size and neurosphere-like circular cells, irregular cells, and cells that have spikes and pseudopodia are observed. Some cells have cell bodies and spikes and/or pseudopodia that are longer than the cell body length (sometimes three times or more). Moreover, it appears that these spikes and/or pseudopodia are attracted to other cells in the culture, and that the cells may be generating an early-stage network and/or cellular junctions among themselves. Images were obtained from x200 magnification of cell cultures.

### Example 4. Colony formation ability of NPCP

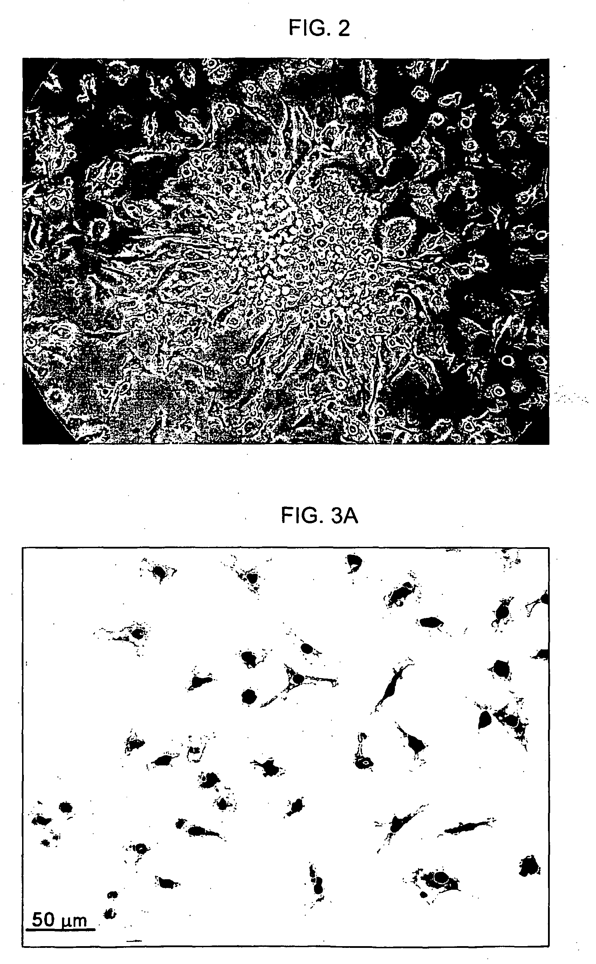

Fig. 2 is a photograph showing the morphology of a CCP-derived NPCP cell cluster, in accordance with an embodiment of the present invention. The ability to generate NPCP cell clusters was tested in the same set of experiments that produced the results shown in Figure 1. A morphological assessment of *in vitro* cluster generation from an NPCP was carried out. The *in vitro* generation of NPCP clusters was carried out in accordance with an embodiment of the present invention, and results are shown in Figure 2. The image in Figure 2 shows typical cluster morphology, and was obtained from x200 magnification of cell cultures.

### Example 5. Cellular immunostaining of the NPCP neural lineage markers

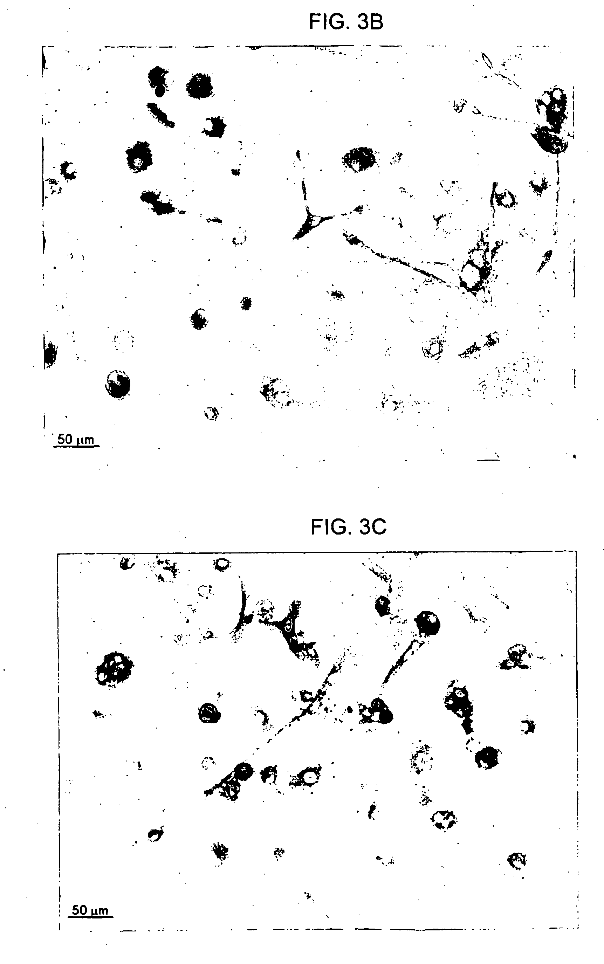

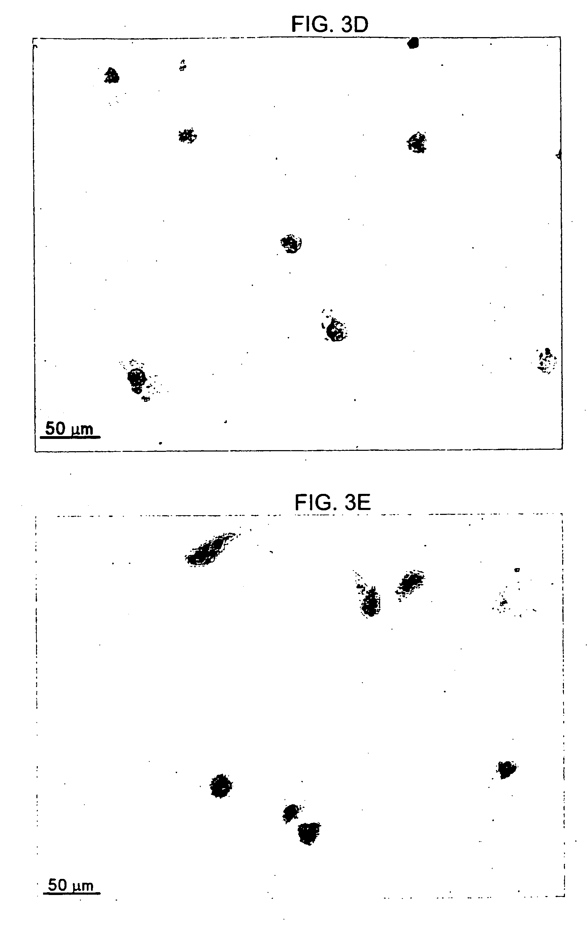

Figs. 3A-E are photographs showing immunostaining results for a CCP-derived NPCP, in accordance with an embodiment of the present invention. In the same set of experiments that produced the results shown in Figure 1, immunostaining of neural lineage markers was performed. Slide-fixed NPCP cells were stained with: Fig. 3A - Anti-Neu-N-Alexa 488; Fig. 3B - Anti-nestin detected by anti-mouse IgG-FITC; Fig. 3C - Anti-tubulin detected by anti-mouse IgG-FITC; Fig. 3D - Anti-04 detected by anti-mouse IgG-Cy3; and Fig. 3E. Anti-GFAP detected by anti-mouse IgG-Cy3. Cells stained with non-specific mouse IgG were detected by anti-mouse IgG-FITC or by anti-mouse IgG-Cy3 used as negative controls.

Peripheral blood was extracted from nine normal human donors for use in nine respective experiments. The *in vitro* generation of the NPCP was carried out in accordance with an embodiment of the present invention. Images in Figure 3 show that NPCP cells expressed Neu-N, a neuron-specific nuclear protein (Figure 3A), and the neural progenitor markers nestin and β-tubulin, expressed by newly differentiated neuronal cells (Figures 3B and 3C). Other cells from these cultures expressed glialspecific antigens, such as 04 and GFAP, which are characteristic of oligodendrocytes and astrocytes, respectively (Figures 3D and 3E). Images were obtained from x100 magnification of slide-fixed cells.

### Example 6. Flow cytometry analysis of the NPCP neuronal markers Nestin and β-Tubulin

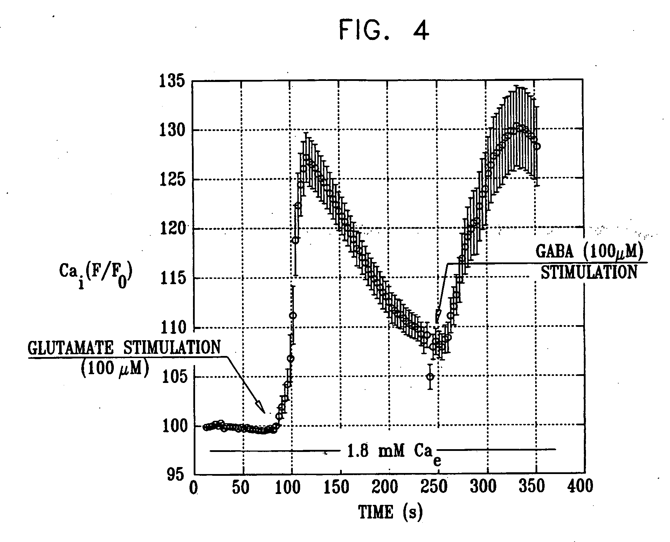

Fig. 4 is a graph showing the percentage of cells testing positive (+/- 1 S.E.) for the neural markers Nestin and β-Tubulin, in accordance with an embodiment of the present invention. In the same set of experiments that produced the results shown in Figures 1, 2 and 3, cellular immunostaining of the neuronal markers Nestin and β-Tubulin was performed at several time points (days 0, 5, 12, 19 and 26) during the culture period. Staining was performed by applying specific antibodies or normal mouse IgG1 (negative isotype control) detected by PE-conjugated anti-mouse antibodies. The in vitro generation of the NPCP was carried out in accordance with an embodiment of the present invention.

The figure shows that Nestin-exhibiting cells can be detected earlier during the culture period (starting form day 5) while β-Tubulin levels are elevated on the cells starting from day 12. The percentages of cells exhibiting β-Tubulin are somewhat lower than Nestin, a phenomenon that might be explained by Nestin being a more general stem cell marker, which is exhibited by other hematopoietic stem cells as well. By day 26, both markers are dramatically reduced. This marker's reduction may indicate that the culture is reorganizing towards more specific cell colonies. (NPCP derived colonies are clearly observed in these cultures; see Figure 2.)

### Example 7. NPCP cells physiologically respond to neurotransmitter stimulation in vitro

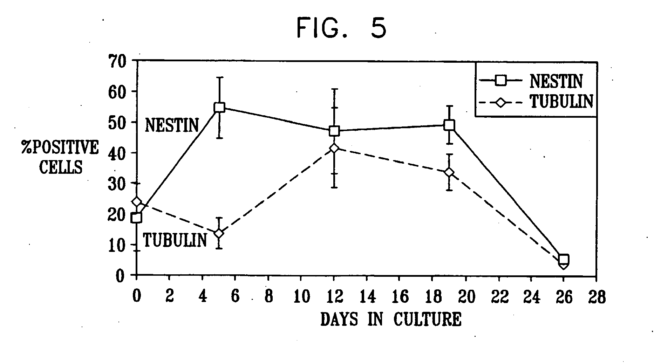

Fig. 5 is a graph showing typical average calcium levels generated by CCP-derived NPCP cells in response to stimulation by the neurotransmitters glutamate and GABA, in accordance with an embodiment of the present invention. In the same set of experiments that produced the results shown in Figures 1-4, NPCP cells' physiological response to neurotransmitters was measured. The in vitro generation of the NPCP was carried out in accordance with an embodiment of the present invention. Ca2+ influx through voltage-gated calcium channels in response to neurotransmitter stimulation with 100 µM glutamate and 100 µM GABA was measured. Harvested cells were loaded with 5 mM Fura-2 acetoxymethyl ester and mounted in a chamber that allowed the superfusion of cells in analytes. Fura-2 was imaged and fluorescent imaging measurements were acquired. The NPCP cells' response to stimulation with the neurotransmitters glutamate and GABA provides evidence for differentiation of CCP cells into neurons.

### Example 8. NPCP cells homing and engraftment in rat model of laser injury in rats' eyes

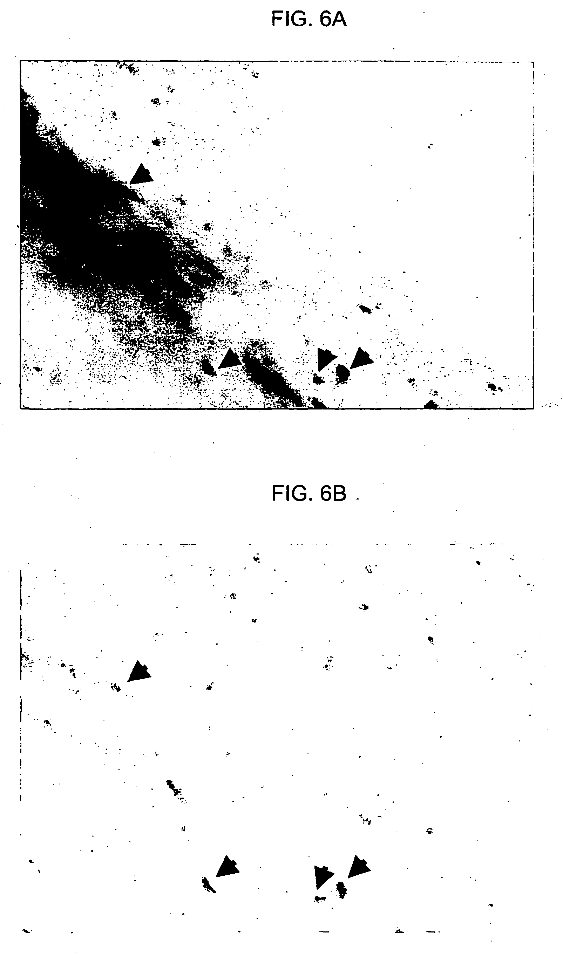

Figs. 6A-B are photographs showing a section of a rat's eye, 20 days after implantation of buman-CCP-derive NPCP cells, in accordance with an embodiment of the present invention.. In part of the same set of experiments that produced the results shown in Figures 1-5, the therapeutic potential of the NPCP cells was assessed. The in vitro generation of the NPCP was carried out in accordance with an embodiment of the present invention. NPCP cells were used for implantation into a rat model of laser-injured retina. Six retinal burns were produced in rat eyes by an argon laser, two disc diameters away from the optic disc. Immediately after lesion induction, 0.45x10^6 NPCP cells were injected into the vitreous cavity of the eye. Paraffin fixated tissue sections were double stained in order to trace engrafted human NPCP cells in the injured retinas. Figure 6 shows a typical section taken from a rat's eye 20 days after human NPCP implantation. Anti-human mitochondria were detected by anti-mouse Cy-3 and with the neuronal nucleic marker anti-Neu-N Alexa 488. Double stained cells are marked by arrows. Slides were counterstained with a fluorescent mounting solution containing the nuclear stain DAPI. Tissue sections stained with non-specific mouse IgG and detected by anti-mouse FITC and by anti-mouse Cy-3 were used as negative controls. Figure 6 shows a tissue section stained with mouse anti-human mitochondria that was detected by anti-mouse Cy-3 (Figure 6A) and with the neuronal nucleic marker anti-Neu-N Alexa 488 (Figure 6B). Double stained cells are marked by arrows. Images were obtained from x100 magnification of tissue section.

The results presented here demonstrate that human NPCP cells can successfully home to damaged retinas as detected by tracing specific engrafted cells in rat eyes.

In accordance with an embodiment of the present invention, extraction of PBMCs is performed using the following protocol:

### Example 1. Extraction of peripheral blood mononuclear cells (PBMC)

Receive blood bag and sterilize it with 70% alcohol
Load blood cells onto a Ficoll gradient.
Spin the tubes for 20 minutes at 1050 g at room temperature (RT), with no brake.
Collect most of the plasma from the upper layer.
Collect the white blood cell fraction from every tube.
Transfer the collected cells to a new 50 ml tube, adjust volume to 30 ml per tube using PBS.
Spin tubes for 15 minutes at 580 g, RT, and discard supernatant.
Count cells in Trypan blue.
Re-suspend in culture medium comprising, for example, X-vivo 15 (TM).

In accordance with an embodiment of the present invention, generation of a CCP is carried out using the following protocol:
Example 1. Generation of a human CCP from PBMCs using a Percoll gradient
Prepare gradient by mixing a ratio of 5.55 Percoll (1.13 g/ml): 3.6 ddH2O: 1 PBSx10.
For every 50 ml tube of Percoll: mix 20 ml of Percoll stock, 13 ml of ddH2O and 3.6 ml of PBSx10.
Mix vigorously, by vortexing, for at least 1 min.
Load 34 ml mix into each 50 ml tube.
Centrifuge tubes, in a fixed angle rotor, for 30 min at 17,000 g, 21°C, with no brake.
Place 150 million - 400 million PBMCs in 3.0 ml medium.
Gently layer 3.0 ml of cell suspension on top of the gradient.
Prepare a second tube with density marker beads: gently layer 3.0 ml of medium on top of the gradient.
Gently load density marker beads - 10 µl from each bead type.
Centrifuge tubes, in a swinging bucket rotor, for 30 min at 1260 g at 13°C, with no brake.
Gently collect all bands located above the red beads.
Centrifuge cells for 15 min at 580 g at 21°C.
Discard supernatant and re-suspend pellet in medium.
Count cells in Trypan blue.
Centrifuge cells for 10 min at 390 g, 21°C.
Discard supernatant and re-suspend pellet in medium.
Take CCP cells for FACS staining.

In accordance with an embodiment of the present invention, the coating of a tissue culture container is carried out using the following protocol:
Culture containers are either un-coated or coated with one or a combination of materials such as collagen, fibronectin, autologous plasma, CD34, BDNF, b-FGF or NGF.

### Example 1. Coating T75 flasks with 25 µg/ml fibronectin and 5 ng/ml BDNF

Prepare 50 ml of 25 µg/ml fibronectin solution in PBS.
Fill every flask with 2-5 ml fibronectin 25 µg/ml.
Incubate at 37°C for at least 30 min.
Collect fibronectin solution.
Wash flask twice in 10 ml PBS.
Prepare 50 ml of 5 ng/ml BDNF solution in PBS.
Fill every flask with 2-5 ml BDNF 10 ng/ml.
Incubate at 37°C for 1 hour.
Collect the solution.
Wash flask twice in 10 ml PBS.

### Example 3. Coating T75 flasks with autologous plasma

Fill every flask with 2-5 ml autologous plasma.
Incubate at 37°C for at least 30 min.
Collect the unbound plasma.
Wash flask twice in 10 ml PBS.

In accordance with an embodiment of the present invention, serum preparation is carried out using the following protocol:
Serum can be obtained directly or prepared from plasma.

### Example 1. Preparation of serum from human plasma:

Take 100 ml plasma
Add 0.5 - 1.0 ml 0.8M CaCl₂-2H₂O for every 50 ml plasma.
Incubate for 0.5 - 3 hours at 37°C.
Spin coagulated plasma 5 min at 2500 g .
Collect the serum in a new tube, avoiding clotting.

In accordance with an embodiment of the present invention, medium preparation is carried out using the following protocol:

Use medium within 10 days from its preparation date. Medium should be serum-free or contain 1-20% autologous serum and/or 1-20% conditioned medium.

Medium can contain one or more additives, such as LIF, EPO, IGF, b-FGF, M-CSF, GM-CSF, TGF alpha, TGF beta, VEGF, BHA, BDNF, B27, F12, NGF, S-100, GDNF, CNTF, EGF, NT3, NT4/5, NGF3, CFN, ADMIF, estrogen, prolactin, an adrenocorticoid, glutamate, serotonin, acetylcholine, NO, retinoic acid (RA), Heparin, insulin, forskolin and/or cortisone, and/or a derivative of any of these, in various concentrations, typically ranging from about 100 pg/ml to about 100 µg/ml (or molar equivalents) and in various volume ratios of about 1:1 to about 1:25 or about 1:25 to about 1:500 of the total medium volume.

### Example 1. Medium for enhancement of CCP-derived NPCP

Serum-free medium (e.g., X-vivo 15 (TM)
10% autologous serum
10 ng/ml bFGF
50 ng/ml NGF
25 ng/ml BDNF

### Example 2. Medium for enhancement of CCP-derived NPCP

Prepare medium as described in Example 1.
Add 200 µM BHA during the last 24 hours of culturing

### Example 3. Medium for enhancement of CCP-derived NPCP

Medium for days 1-5:
Serum-free medium (e.g., X-vivo 15 (TM))
10% autologous serum
5 IU/ml Heparin
10 ng/ml bFGF
50 ng/ml NGF
25 ng/ml BDNF

Defined medium for days 5-30:
Serum-free medium / (e.g., X-vivo 15 (TM)
5 IU/ml Heparin
33.3% F12
2% B27
20 ng/ml bFGF
20 ng/ml EGF

### Example 4. Medium for enhancement of CCP-derived NPCP

Medium for days 1-5:
Serum-free medium (e.g., X-vivo 15 (TM))
10% autologous serum
5 IU/ml Heparin

Defined medium for days 5-30:
Serum-free medium / (e.g., X-vivo 15 (TM)
33.3% F12
4% B27
20 ng%ml bFGF
20 ng/ml EGF

In accordance with an embodiment of the present invention, culturing of a CCP to produce a NPCP is carried out using the following protocol:

### Example 1. Culturing of CCP cell suspension in T75 Flasks.

Spin suspension for 15 minutes at 450 g, 21°C.
Discard the supernatant.
Re-suspend pellet to 1-2.5 million CCP cells / ml.
Seed cells in T75 flasks
Incubate T75 flasks, plates and slides at 37°C, 5% CO2.

### Example 2. Reseeding of adherent and/or detached and/or floating cells

For some applications, increased expansion and differentiation of the CCP may be achieved by re-seeding collected cells on new pre-coated dishes in culture medium.
Collect all cultured CCP.
Spin tubes for 10 minutes at 450 g, 21 °C.
Discard the supernatant.
Re-suspend cells in culture medium and seed in a new pre-coated T75 flasks.

Continue culturing the cells, and perform all other activities (e.g., medium refreshment, visual inspection, and/or flow cytometry), as appropriate, as described herein.

This procedure can be performed weekly during the culture period and/or within 24, 48, or 72 hours before termination of the culture.

In accordance with an embodiment of the present invention, refreshing of the media in ongoing growing CCP cultures is carried out using the following protocol:

Refreshing of the media in ongoing growing flasks should occur every 3-4 days.

### Example 1. Refreshing of medium in T-75 Flasks.

Collect non-adherent cells in 50 ml tubes.

Fill every flask with 10 ml fresh culture medium enriched with conditioned medium.

Spin tubes for 10 minutes at 450 g, RT; discard the supernatant.

Gently mix cell pellet, re-suspend in 10 ml/flask fresh culture medium and return to the flasks.

In accordance with an embodiment of the present invention, harvesting of resulted NPCP is carried out using the following protocol:

### Example 1. Collection of enriched NPCP cultures.

Collect non-adherent cells in 50 ml tubes.

Wash flask surface by pipetting with cold PBS.

Add 5 ml of cold PBS.

Detach remaining adherent cells using a scraper and if needed, 5 ml EDTA.

Collect the detached cells and add them to the tube.

Spin tube for 5 min, at 450 g, room temperature.

Re-suspend the pellet in 2-5 ml PBS.

Count the cells.

In accordance with an embodiment of the present invention, cellular product preservation is carried out using the following protocol:

Cellular product can be kept in preservation media or frozen in freezing buffer until use for transplantation into a patient.

### Example 1. Cryopreservation of NPCP

Prepare freezing buffer containing 90% human autologous serum and 10% DMSO.

### Example 2. Short-period preservation of NPCP

Prepare preservation medium including growth medium containing 1-20% autologous serum with few or no other additives.

In accordance with an embodiment of the present invention, FACS Staining is carried out using the following protocol:

### Example 1.

FACS staining protocol for fixed permeabilized cells:

| Tube No. | Staining 1^{st} step | Staining 2^{nd} step | Aim of staining |
|---|---|---|---|
| 1. | - | - | Un-stained control |
| 2. | CD45-FITC | - | Single staining for PMT and compensation settings |
| 3: | CD45-PE | - | |
| 4. | mIgG1FITC | - | Isotype control |
| 5. | mIgG1 | Anti mouse-PE | Isotype control |
| 6. | Neu-N-Alexa488 | | |
| 7. | Nestin | Anti mouse-PE | |
| 8. | β-Tubulin | Anti mouse-PE | |
| 9. | 04 | Anti mouse-PE | |
| 10. | GFAP | Anti mouse-PE | |

In accordance with an embodiment of the present invention, immunofluorescent staining is carried out using the following protocol:

### Example 1.

Cellular staining of slide fixed NPCP

Staining protocol

| Slide No. | Staining 1^{st} step | Staining 2^{nd} step | Aim of staining |
|---|---|---|---|
| 1. | - | NeuN-Alexa488 | Isotype control |
| 2. | -mIgG1 | mIgG1-FITC | |
| 3. | mIgG1 | Anti mouse-PE | Isotype control |
| 4. | Nestin | Anti mouse-FITC | |
| 5. | β-Tubulin | Anti mouse-FITC | |
| 6. | GFAP | Anti mouse-PE | |
| 7. | 04 | Anti mouse-cy3 | |

In vitro testing of NPCP physiological response to neurotransmitters

### Example 1. Calcium Uptake Assay

Ca2+ influx through voltage-gated calcium channels in response to neurotransmitter stimulation with 100 µM glutamate and 100 µM GABA (Sigma-Aldrich), was performed as described herein

Harvested cells were cultured overnight on 33 mm glass slides coated with poly-L-lysine.

Cells were incubated for 30 minutes with 5 mM Fura-2 acetoxymethyl ester (AM; TEF-Lab) in 0.1% BSA in NaCl Ringer's solution.

After dye loading, the cells were washed in Ringer's solution, and the cover slides were mounted in a chamber that allowed the superfusion of cells.

Fura-2 was excited at 340 nm and 380 mm and imaged with a 510-nm long-pass filter. The imaging system consisted of an Axiovert 100 inverted microscope (Zeiss), Polychrome II monochromator (TILL Photonics), and a SensiCam cooled charge-coupled device (PCO).

Fluorescent imaging measurements were acquired with Imaging Workbench 2 (Axon Instruments). (See Hershfinkel M, Moran A, Grossman N et al. A zinc-sensing receptor triggers the release of intracellular Ca2+ and regulates ion transport. Proc Natl Acad Sci USA 2001;98:11749-11754)

For some applications, techniques described herein are practiced in combination with techniques described in one or more of the references cited in the present patent application.

It is to be appreciated that by way of illustration and not limitation, techniques are described herein with respect to cells derived from an animal source.

## Claims

1. A method comprising in vitro stimulating a core cell population (CCP) derived from peripheral blood of a donor, wherein the CCP comprises at least 5 million cells that have a density of less than 1.072 g/ml, and at least 1.5% of which are CD34+CD45-/dim, with growth factors to differentiate into a neural progenitor/precursor cell population (NPCP) wherein:
at least some of the cells of the neural progenitor/precursor cell population express Neu-N,
at least some of the cells of the neural progenitor/precursor cell population express nestin,
at least some of the cells of the neural progenitor/precursor cell population express beta-tubulin,
at least some of the cells of the neural progenitor/precursor cell population express 04, and
at least some of the cells of the neural progenitor/precursor cell population express GFAP.

2. The method according to claim 1, wherein at least 1.5 million of the cells in the NPCP and at least 1.5% of the cells in the NPCP comprise at least one molecule or molecular structure selected from the list consisting of: CD34, CD117, CD44, Neu-N, nestin, microtubule associated protein-1 (MAP-1), MAP Tau, microtubule associated protein-2 (MAP-2), neurofilament NF200, neuron-specific enolase (NSE), choline acetyltransferase (ChAT) neuronal class III β-Tubulin, glutamic acid decarboxylase (GAD), S-100, GDNF, CNTF, BDNF, NGF, NT3, NT4/5, glutamic acid, gamma-aminobutyric acid (GABA), oligodendrocyte marker (04), myelin basic protein (MBP), galactocerebroside (GalC), glial fibrillary acidic protein (GFAP), CD211 b, dopamine, norepinephrine, epinephrine, glutamate, glycine, acetylcholine, serotonin, and endorphin.

3. The method according to claim 1, further comprising preparing the NPCP as a research tool.

4. The method according to claim 1, wherein the CCP is derived by applying cells extracted from a mammalian donor to one or more density gradients suitable for selecting cells having a density less than 1.072 g/ml.

5. The method according to claim 1, wherein the CCP is **characterized by** at least 40% of the CCP being CD31 +.

6. The method according to claim 1, wherein stimulating the CCP comprises stimulating the CCP to differentiate into a pre-designated, desired class of neural progenitor cells.

7. The method according to claim 1, wherein the CCP is derived from frozen tissue.

8. The method according to claim 1, wherein stimulating the CCP comprises incubating the CCP in a container having a surface comprising at least one of: an antibody and autologous plasma.

9. The method according to claim 1, further comprising freezing the CCP prior to stimulating the CCP.

10. The method according to claim 1, further comprising freezing the NPCP.

11. The method according to claim 1, further comprising transporting the CCP to a site at least 10 km from a site where the CCP is first created, and stimulating the CCP at the remote site.

12. The method according to claim 1, further comprising transporting the NPCP to a site at least 10 km from a site where the NPCP is first created.

13. The method according to claim 1, further comprising preparing the NPCP as a product for administration to a patient.

14. The method according to claim 1, further comprising in vitro facilitating a diagnosis responsive to stimulating the CCP to differentiate into the NPCP.

15. The method according to claim 1, wherein stimulating the CCP comprises incubating the CCP in a container with a surface comprising a growth-enhancing molecule other than collagen or fibronectin.

16. The method according to claim 15, further comprising applying to the surface a layer that includes the growth-enhancing molecule and a separate layer that includes at least one of: collagen, fibronectin, and autologous plasma.

17. The method according to claim 1, wherein stimulating comprises culturing the CCP with at least one factor derived from a target tissue.

18. The method according to claim 1, wherein stimulating comprises co-culturing the CCP with a target tissue.

19. The method according to claim 1, wherein at least some of the cells of the neural progenitor/precursor cell population express Neu-N, nestin, and beta-tubulin.

## Patentansprüche

1. Verfahren, umfassend die in vitro-Stimulation einer Kern-Zellpopulation (CCP), die aus dem peripheren Blut eines Donors stammt, wobei die CCP mindestens 5 Millionen Zellen umfasst, die eine Dichte von weniger als 1,072 g/ml aufweisen, und von denen mindestens 1,5% CD34+CD45-/dim sind, mit Wachstumsfaktoren, um zu einer neuralen Vorläufer-/Vorstufen-Zellpopulation (NPCP) zu differentieren, wobei:
zumindest einige der Zellen der neuralen Vorläufer-/Vorstufen-Zellpopulation Neu-N exprimieren,
zumindest einige der Zellen der neuralen Vorläufer-/Vorstufen-Zellpapulation Nestin exprimieren,
zumindest einige der Zellen der neuralen Vorläufer-Norstufen-Zellpopulation beta-Tubulin exprimieren,
zumindest einige der Zellen der neuralen Vorläufer-/Vorstufen-Zellpopulation 04 exprimieren und
zumindest einige der Zellen der neuralen Vorläufer-/Vorstufen-Zellpopulation GFAP exprimieren.

2. Verfahren gemäß Anspruch 1, wobei mindestens 1,5 Millionen der Zellen in der NPCP und mindestens 1,5% der Zellen in der NPCP mindestens ein Molekül oder eine Molekülstruktur, ausgewählt aus der Liste, bestehend aus: CD34, CD117, CD44, Neu-N, Nestin, Mikrotubuli-assoziiertem Protein-1 (MAP-1), MAP-Tau, Mikrotubuli-assoziiertem Protein-2 (MAP-2), Neurofilament NF200, neuronenspezifischer Enolase (NSE), Cholinacetyltransferase (ChAT), neuronalem Klasse III β-Tubulin, Glutaminsäuredecarboxylase (GAD), S-100, GDNF, CNTF, BDNF, NGF, NT3, NT4/5, Glutaminsäure, gamma-Aminobuttersäure (GABA), Oligodendrozytenmarker (04), Myelin-Basischem Protein (MBP), Galactocerebrosid (GalC), saurem Gliafaserprotein (GFAP), CD211 b, Dopamin, Norepinephrin, Epinephrin, Glutamat, Glycin, Acetylcholin, Serotonin und Endorphin, umfassen.

3. Verfahren gemäß Anspruch 1, welches weiterhin das Vorbereiten der NPCP als Forschungswerkzeug umfasst.

4. Verfahren gemäß Anspruch 1, wobei die CCP erhalten wird, indem auf Zellen, die aus einem Säugetier-Donor extrahiert wurden, ein oder mehrere Dichtegradient(en), der/die für die Selektion von Zellen, die eine Dichte von weniger als 1,072 g/ml aufweisen, geeignet ist/sind, angelegt wird/werden.

5. Verfahren gemäß Anspruch 1, wobei die CCP **dadurch gekennzeichnet ist, dass** mindestens 40% der CCP CD31 + sind.

6. Verfahren gemäß Anspruch 1, wobei die Stimulation der CCP die Stimulation der CCP, damit sie in eine vorbestimmte, gewünschte Klasse neuraler Vorläuferzellen differenziert, umfasst.

7. Verfahren gemäß Anspruch 1, wobei die CCP aus gefrorenem Gewebe stammt.

8. Verfahren gemäß Anspruch 1, wobei die Stimulation der CCP das Inkubieren der CCP in einem Behälter umfasst, der eine Oberfläche aufweist, die mindestens eines von einem Antikörper und autologem Plasma umfasst.

9. Verfahren gemäß Anspruch 1, welches weiterhin das Einfrieren der CCP vor der Stimulation der CCP umfasst.

10. Verfahren gemäß Anspruch 1, welches weiterhin das Einfrieren der NPCP umfasst.

11. Verfahren gemäß Anspruch 1, welches weiterhin den Transport der CCP zu einem Ort, der mindestens 10 km von einem Ort entfernt ist, an dem die CCP zuerst erzeugt wurde, und die Stimulation der CCP an dem entfernten Ort umfasst.

12. Verfahren gemäß Anspruch 1, welches weiterhin den Transport der NPCP zu einem Ort, der mindestens 10 km von einem Ort entfernt ist, an dem die NPCP zuerst erzeugt wurde, umfasst.

13. Verfahren gemäß Anspruch 1, welches weiterhin das Vorbereiten der NPCP als Produkt für die Verabreichung an einen Patienten umfasst.

14. Verfahren gemäß Anspruch 1, welches weiterhin umfasst, eine Diagnose, die auf die Stimulation der CCP, damit sie zu der NPCP differenziert, anspricht, in vitro zu erleichtern.

15. Verfahren gemäß Anspruch 1, wobei die Stimulation der CCP das Inkubieren der CCP in einem Behälter mit einer Oberfläche, die ein wachstumsförderndes Molekül, das nicht Kollagen oder Fibronectin ist, umfasst.

16. Verfahren gemäß Anspruch 15, welches weiterhin das Aufbringen einer Schicht, die das wachstumsfördernde Molekül umfasst, und einer separaten Schicht, die mindestens eines von Kollagen, Fibronectin und autologem Plasma umfasst, auf die Oberfläche umfasst.

17. Verfahren gemäß Anspruch 1, wobei die Stimulation das Kultivieren der CCP mit mindestens einem Faktor, der aus einem Ziel-Gewebe stammt, umfasst.

18. Verfahren gemäß Anspruch 1, wobei die Stimulation die Co-Kultivierung der CCP mit einem Ziel-Gewebe umfasst.

19. Verfahren gemäß Anspruch 1, wobei zumindest einige der Zellen der neuralen Vorläufer-/Vorstufen-Zellpopulation Neu-N, Nestin und beta-Tubulin exprimieren.

## Revendications

1. Méthode comprenant la stimulation in vitro d'une population de cellules centrales (CCP) provenant du sang périphérique d'un donneur, dans laquelle la CCP comprend au moins 5 millions de cellules qui ont une masse volumique inférieure à 1,072 g/ml, et dont une proportion d'au moins 1,5 % est du type CD34+CD45-/dim, avec des facteurs de croissance pour la différentiation en une population de cellules parentales/précurseurs neurales (NPCP), où :
au moins certaines des cellules de la population de cellules parentales/précurseurs neurales expriment Neu-N,
au moins certaines des cellules de la population de cellules parentales/précurseurs neurales expriment de la nestine ;
au moins certaines des cellules de la population de cellules parentales/précurseurs neurales expriment de la bêta-tubuline ;
au moins certaines des cellules de la population de cellules parentales/précurseurs neurales expriment 04, et
au moins certaines des cellules de la population de cellules parentales/précurseurs neurales expriment la GFAP.

2. Méthode suivant la revendication 1, dans laquelle au moins 1,5 million des cellules dans la NPCP et au moins 1,5 % des cellules dans la NPCP comprennent au moins une molécule ou structure moléculaire choisie dans la liste consistant en : CD34, CD117, CD44, Neu-N, la nestine, la protéine associée aux microtubules (MAP-1), MAP Tau, la protéine-2 associée aux microtubules (MAP-2), la NF200 des neurofilaments, l'énolase spécifique des neurones (NSE), la choline-acétyltransférase (ChAT), la β-tubuline neuronale de classe III, l'acide glutamique-décarboxylase (GAD), S-100, GDNF, CNTF, BDNF, NGF, NT3, NT4/5, l'acide glutamique, l'acide gamma-aminobutyrique (GABA), le marqueur des oligodendrocytes (04), la protéine basique de la myéline (MBP), le galactocérébroside (GalC), la protéine acide fibrillaire gliale (GFAP), CD211b, la dopamine, la norépinéphrine, l'épinéphrine, le glutamate, la glycine, l'acétylcholine, la sérotonine et l'endorphine.

3. Méthode suivant la revendication 1, comprenant en outre la préparation de la NPCP comme outil de recherche.

4. Méthode suivant la revendication 1, dans laquelle la CCP est obtenue en appliquant les cellules extraites d'un mammifère servant de donneur, à un ou plusieurs gradients de densité convenables pour la sélection de cellules ayant une masse volumique inférieure à 1,072 g/ml.

5. Méthode suivant la revendication 1, dans laquelle la CCP est **caractérisée par** au moins une proportion d'au moins 40 % de la cCP consistant en CD31+.

6. Méthode suivant la revendication 1, dans laquelle la stimulation de la CCP comprend la stimulation de la CCP pour la différentiation en une classe désirée, prédésignée, de cellules parentales neurales.

7. Méthode suivant la revendication 1, dans laquelle la CCP est dérivée d'un tissu congelé.

8. Méthode suivant la revendication 1, dans laquelle la stimulation de la CCP comprend la mise en incubation de la CCP dans un récipient ayant une surface comprenant au moins une des entités : un anticorps et du plasma autologue.

9. Méthode suivant la revendication 1, comprenant en outre la congélation de la CCP avant stimulation de la CCP.

10. Méthode suivant la revendication 1, comprenant en outre la congélation de la NPCP.

11. Méthode suivant la revendication 1, comprenant en outre le transport de la CCP à un site situé à au moins 10 km d'un site où la CCP est créée initialement, et la stimulation de la CCP au site éloigné.

12. Méthode suivant la revendication 1, comprenant en outre le transport de la NPCP à un site situé à au moins 10 km d'un site où la NPCP est créée initialement.

13. Méthode suivant la revendication 1, comprenant en outre la préparation de la NPCP comme produit pour l'administration à un patient.

14. Méthode suivant la revendication 1, comprenant en outre la facilitation in vitro d'un diagnostic sensible à la stimulation de la CCP pour la différentiation en la NPCP.

15. Méthode suivant la revendication 1, dans laquelle la stimulation de la CCP comprend la mise en incubation de la CCP dans un récipient avec une surface comprenant une molécule activatrice de croissance autre que le collagène ou la fibronectine.

16. Méthode suivant la revendication 15, comprenant en outre l'application à la surface d'une couche qui comprend la molécule activatrice de croissance et d'une couche distincte qui comprend au moins une des entités : le collagène, la fibronectine et du plasma autologue.

17. Méthode suivant la revendication 1, dans laquelle la stimulation comprend la culture de la CCP avec au moins un facteur dérivé d'un tissu cible.

18. Méthode suivant la revendication 1, dans laquelle la stimulation comprend la co-culture de la CCP avec un tissu cible.

19. Méthode suivant la revendication 1, dans laquelle au moins certaines des cellules de la population de cellules parentales/précurseurs neurales expriment la Neu-N, la nestine et la bêta-tubuline.
